# EUROPEAN PATENT APPLICATION

(11) **EP 4 345 166 A2**
(43) Date of publication of application: **03.04.2024**
(21) Application number: 23200019.0
(22) Date of filing: 27.09.2023
(51) Int. Cl.: C12P 13/10, C12N 9/88, C12P 13/14, C12P 13/24

(54) **METHOD FOR PRODUCING L-AMINO ACID**

(30) Priority: 30.09.2022 JP 2022157223; 12.07.2023 JP 2023114218
(71) Applicant: AJINOMOTO CO., INC., Chuo-ku Tokyo 104-8315 (JP)
(72) Inventor: HAMANO, Chie, Kanagawa, 210-8681 (JP); ITO, Yoshihiro, Kanagawa, 210-8681 (JP); EBARA, Naoki, Kanagawa, 210-8681 (JP); INOUE, Kota, Kanagawa, 210-8681 (JP); ONO, Yukiko, Kanagawa, 210-8681 (JP); ISHIDA, Rihito, Kanagawa, 210-8681 (JP); ONISHI, Fumito, Kanagawa, 210-8681 (JP); HARA, Yoshihiko, Kanagawa, 210-8681 (JP)
(74) Representative: Strehl Schübel-Hopf & Partner

(57) **Abstract**

A method for producing an L-amino acid such as L-glutamic acid is provided. An L-amino acid is produced by culturing in a culture medium a bacterium belonging to the family Enterobacteriaceae and having an L-amino acid-producing ability, and collecting the L-amino acid from the culture medium and/or cells of the bacterium, wherein the bacterium has been modified to have one or more modifications selected from the modifications (A) to (F) shown below:
(A) modification of reducing the activity of a BudA protein;
(B) modification of reducing the activity of a BudB protein;
(C) modification of reducing the activity of a BudC protein;
(D) modification of reducing the activity of a PAJ_3461 protein;
(E) modification of reducing the activity of a PAJ 3462 protein; and
(F) modification of reducing the activity of a PAJ_3463 protein.

## Description

### Technical Field

The present invention relates to a method for producing an L-amino acid such as L-glutamic acid by fermentation using a bacterium. L-Amino acids are industrially useful as raw materials of seasonings and so forth.

### Background Art

L-Amino acids are industrially produced by, for example, fermentation using microorganisms such as bacteria having an L-amino acid-producing ability (Non-patent document 1). As such microorganisms, for example, strains isolated from the nature and mutant strains thereof have been used. Also, an L-amino acid-producing ability of microorganisms can be improved using recombinant DNA techniques.

A BudA protein encoded by a budA gene is known as acetolactate decarboxylase. A BudB protein encoded by a budB gene is known as acetolactate synthase. A BudC protein encoded by a budC gene is known as (S,S)-butanediol dehydrogenase, meso-2,3-butandiol dehydrogenase, or diacetyl reductase.

APAJ_3461 protein, a PAJ_3462 protein, and a PAJ_3463 protein encoded by a PAJ_3461 gene, a PAJ_3462 gene, and a PAJ_3463 gene are known as respective subunits constituting gluconate 2-dehydrogenase.

A technique of producing substances such as 2-oxogluterate from carbon monoxide as a carbon source using a microorganism having a reduced or deleted 2,3-butandiol producing ability (Patent document 1).

### Prior art references

### Patent documents

Patent document 1: WO2013/115659

### Non-patent documents

Non-patent document 1: Akashi, K. et al., Amino Acid Fermentation. Japan Scientific Societies Press, p.195 to 215, 1986.

### Summary of the Invention

### Object to be Achieved by the Invention

An object of the present invention is to develop a novel technique for improving an L-amino acid-producing ability of a bacterium, and thereby provide a method for efficiently producing an L-amino acid.

### Means for Achieving the Object

In order to achieve the aforementioned object, the inventors of the present invention conducted various researches. As a result, they found that an L-amino acid-producing ability of a bacterium belonging to the family Enterobacteriaceae can be improved by modifying the bacterium to have one or more modifications selected from the modifications (A) to (F) shown below, and they accomplished the present invention:
(A) modification of reducing the activity of a BudA protein;
(B) modification of reducing the activity of a BudB protein;
(C) modification of reducing the activity of a BudC protein;
(D) modification of reducing the activity of a PAJ_3461 protein;
(E) modification of reducing the activity of a PAJ_3462 protein; and
(F) modification of reducing the activity of a PAJ_3463 protein.

The present invention can be thus embodied, for example, as follows.
[1] A method for producing an L-amino acid, the method comprising:
   culturing a bacterium belonging to the family Enterobacteriaceae and having an L-amino acid-producing ability in a culture medium to accumulate the L-amino acid in the culture medium and/or cells of the bacterium; and
   collecting the L-amino acid from the culture medium and/or the cells,
   wherein the L-amino acid is an L-amino acid of glutamate family, and
   wherein the bacterium has one or more modifications selected from the modifications (A) to (F) shown below:
      (A) modification of reducing the activity of a BudA protein;
      (B) modification of reducing the activity of a BudB protein;
      (C) modification of reducing the activity of a BudC protein;
      (D) modification of reducing the activity of a PAJ_3461 protein;
      (E) modification of reducing the activity of a PAJ 3462 protein; and
      (F) modification of reducing the activity of a PAJ_3463 protein.
[2] The method mentioned above (specifically, according to [1]), wherein the bacterium at least has one or more modifications selected from the modifications (A) to (C).
[3] The method mentioned above (specifically, according to [1] or [2]), wherein the bacterium at least has the modifications (A) to (C).
[4] The method mentioned above (specifically, according to any one of [1] to [3]), wherein the bacterium at least has one or more modifications selected from the modifications (D) to (F).
[5] The method mentioned above (specifically, according to any one of [1] to [4]), wherein the bacterium at least has the modifications (D) to (F).
[6] The method mentioned above (specifically, according to any one of [1] to [5]), wherein the bacterium has the modifications (A) to (F).
[7] The method mentioned above (specifically, according to any one of [1] to [6]),
   Wherein the activity of the BudA protein is reduced by reducing the expression of a budA gene and/or disrupting the budA gene;
   Wherein the activity of the BudB protein is reduced by reducing the expression of a budB gene and/or disrupting the budB gene;
   Wherein the activity of the BudC protein is reduced by reducing the expression of a budC gene and/or disrupting the budC gene;
   Wherein the activity of the PAJ_3461 protein is reduced by reducing the expression of a PAJ_3461 gene and/or disrupting the PAJ_3461 gene;
   Wherein the activity of the PAJ_3462 protein is reduced by reducing the expression of a PAJ_3462 gene and/or disrupting the PAJ_3462 gene; and/or
   Wherein the activity of the PAJ_3463 protein is reduced by reducing the expression of a PAJ_3463 gene and/or disrupting the PAJ_3463 gene.
[8] The method mentioned above (specifically, according to [7]),
   wherein the expression of the budA gene is reduced by modifying an expression control sequence of the budA gene;
   wherein the expression of the budB gene is reduced by modifying an expression control sequence of the budB gene;
   wherein the expression of the budC gene is reduced by modifying an expression control sequence of the budC gene;
   wherein the expression of the PAJ_3461 gene is reduced by modifying an expression control sequence of the PAJ_3461 gene;
   wherein the expression of the PAJ_3462 gene is reduced by modifying an expression control sequence of the PAJ_3462 gene; and/or
   wherein the expression of the PAJ_3463 gene is reduced by modifying an expression control sequence of the PAJ_3463 gene.
[9] The method mentioned above (specifically, according to any one of [1] to [8]),
   Wherein the activity of the BudA protein is reduced by deletion of the budA gene;
   Wherein the activity of the BudB protein is reduced by deletion of the budB gene;
   Wherein the activity of the BudC protein is reduced by deletion of the budC gene;
   Wherein the activity of the PAJ_3461 protein is reduced by deletion of the PAJ_3461 gene;
   Wherein the activity of the PAJ_3462 protein is reduced by deletion of the PAJ_3462 gene; and/or
   Wherein the activity of the PAJ_3463 protein is reduced by deletion of the PAJ_3463 gene.
[10] The method mentioned above (specifically, according to any one of [1] to [9]),
   wherein the BudA protein is the protein (1a), (1b), or (1c) shown below:
      (1a) a protein comprising the amino acid sequence of SEQ ID NO: 2;
      (1b) a protein comprising the amino acid sequence of SEQ ID NO: 2, but which includes substitution, deletion, insertion, and/or addition of 1 to 10 amino acid residues, and having acetolactate decarboxylase activity;
      (1c) a protein comprising an amino acid sequence showing an identity of 90% or higher to the amino acid sequence of SEQ ID NO: 2, and having acetolactate decarboxylase activity;
   wherein the BudB protein is the protein (2a), (2b), or (2c) shown below:
      (2a) a protein comprising the amino acid sequence of SEQ ID NO: 4;
      (2b) a protein comprising the amino acid sequence of SEQ ID NO: 4, but which includes substitution, deletion, insertion, and/or addition of 1 to 10 amino acid residues, and having acetolactate synthase activity;
      (2c) a protein comprising an amino acid sequence showing an identity of 90% or higher to the amino acid sequence of SEQ ID NO: 4, and having acetolactate synthase activity;
   wherein the BudC protein is the protein (3a), (3b), or (3c) shown below:
      (3a) a protein comprising the amino acid sequence of SEQ ID NO: 6;
      (3b) a protein comprising the amino acid sequence of SEQ ID NO: 6, but which includes substitution, deletion, insertion, and/or addition of 1 to 10 amino acid residues, and having (S,S)-butanediol dehydrogenase activity, meso-2,3-butandiol dehydrogenase activity, and/or diacetyl reductase activity;
      (3c) a protein comprising an amino acid sequence showing an identity of 90% or higher to the amino acid sequence of SEQ ID NO: 6, and having (S,S)-butanediol dehydrogenase activity, meso-2,3-butandiol dehydrogenase activity, and/or diacetyl reductase activity;
   wherein the PAJ_3461 protein is the protein (4a), (4b), or (4c) shown below:
      (4a) a protein comprising the amino acid sequence of SEQ ID NO: 8;
      (4b) a protein comprising the amino acid sequence of SEQ ID NO: 8, but which includes substitution, deletion, insertion, and/or addition of 1 to 10 amino acid residues, and having gluconate 2-dehydrogenase activity;
      (4c) a protein comprising an amino acid sequence showing an identity of 90% or higher to the amino acid sequence of SEQ ID NO: 8, and having gluconate 2-dehydrogenase activity;
   wherein the PAJ_3462 protein is the protein (5a), (5b), or (5c) shown below:
      (5a) a protein comprising the amino acid sequence of SEQ ID NO: 10;
      (5b) a protein comprising the amino acid sequence of SEQ ID NO: 10, but which includes substitution, deletion, insertion, and/or addition of 1 to 10 amino acid residues, and having gluconate 2-dehydrogenase activity;
      (5c) a protein comprising an amino acid sequence showing an identity of 90% or higher to the amino acid sequence of SEQ ID NO: 10, and having gluconate 2-dehydrogenase activity; and/or
   wherein the PAJ_3463 protein is the protein (6a), (6b), or (6c) shown below:
      (6a) a protein comprising the amino acid sequence of SEQ ID NO: 12;
      (6b) a protein comprising the amino acid sequence of SEQ ID NO: 12, but which includes substitution, deletion, insertion, and/or addition of 1 to 10 amino acid residues, and having gluconate 2-dehydrogenase activity;
      (6c) a protein comprising an amino acid sequence showing an identity of 90% or higher to the amino acid sequence of SEQ ID NO: 12, and having gluconate 2-dehydrogenase activity.
[11] The method mentioned above (specifically, according to any one of [1] to [10]), wherein the bacterium is a Pantoea bacterium or an Escherichia bacterium.
[12] The method mentioned above (specifically, according to any one of [1] to [11]), wherein the bacterium is Pantoea ananatis or Escherichia coli.
[13] The method mentioned above (specifically, according to any one of [1] to [12]), wherein the L-amino acid of glutamate family is one or more L-amino acids selected from the group consisting of L-glutamic acid, L-glutamine, L-proline, L-arginine, L-citrulline, and L-omithine.
[14] The method mentioned above (specifically, according to any one of [1] to [13]), wherein the L-amino acid of glutamate family is L-glutamic acid.
[15] The method mentioned above (specifically, according to [13] or [14]), wherein the L-glutamic acid is ammonium L-glutamate or sodium L-glutamate.

According to the present invention, an L-amino acid-producing ability of a bacterium can be improved, and an L-amino acid can be efficiently produced.

### Modes for Carrying out the Invention

Hereinafter, the present invention will be explained in detail.

The method of the present invention is a method for producing an L-amino acid comprising culturing a bacterium belonging to the family Enterobacteriaceae and having an L-amino acid-producing ability in a culture medium to accumulate the L-amino acid in the culture medium and/or cells of the bacterium, and collecting the L-amino acid from the culture medium and/or cells of the bacterium, wherein the bacterium has been modified to have a specific feature. The bacterium used for this method is also referred to as "bacterium of the present invention".

### <1> Bacterium of the present invention

The bacterium of the present invention is a bacterium belonging to the family Enterobacteriaceae and having an L-amino acid-producing ability, which has been modified to have the specific feature.

### <1-1> Bacterium having L-amino acid-producing ability

In the present invention, the phrase "bacterium having an L-amino acid-producing ability" refers to a bacterium having an ability to generate and accumulate an objective L-amino acid in a culture medium and/or cells of the bacterium in such a degree that the L-amino acid can be collected, when the bacterium is cultured in the culture medium. The bacterium having the L-amino acid-producing ability may be a bacterium that is able to accumulate an objective L-amino acid in a culture medium and/or cells of the bacterium in an amount larger than that obtainable with a non-modified strain. The phrase "non-modified strain" refers to a control strain that has not been modified to have the specific feature. That is, examples of the non-modified strain include a wild-type strain and parental strain. The bacterium having the L-amino acid-producing ability may be a bacterium that is able to accumulate an objective L-amino acid in a culture medium in an amount of preferably 0.5 g/L or more, more preferably 1.0 g/L or more.

The L-amino acid to be produced in the present invention is an L-amino acid of glutamate family. The phrase "L-amino acid of glutamate family" collectively refers to L-glutamic acid and L-amino acids that are biosynthesized via L-glutamic acid as an intermediate. Examples of the L-amino acids that are biosynthesized via L-glutamic acid as an intermediate include L-glutamine, L-proline, L-arginine, L-citrulline, and L-ornithine. Particular examples of the L-amino acid of glutamate family include L-glutamic acid. The bacterium of the present invention may have an ability to produce a single kind of L-amino acid or two or more kinds of L-amino acids.

In the present invention, the phrase "amino acid" refers to an L-amino acid, unless otherwise stated. In the present invention, the phrase "L-amino acid" refers to an L-amino acid in a free form, a salt thereof, or a mixture thereof, unless otherwise stated. Examples of the salt will be described below.

Examples of bacteria belonging to the family Enterobacteriaceae include bacteria belonging to the genus Escherichia, Enterobacter, Pantoea, Klebsiella, Serratia, Erwinia, Photorhabdus, Providencia, Salmonella, Morganella, or the like. Specifically, bacteria classified into the family Enterobacteriaceae according to the taxonomy used in the NCBI (National Center for Biotechnology Information) database (http://www.ncbi.nlm.nih.gov/Taxonomy/Browser/wwwtax.cgi?id=91347) can be used.

The Escherichia bacteria are not particularly limited, and examples thereof include those classified into the genus Escherichia according to the taxonomy known to those skilled in the field of microbiology. Examples of the Escherichia bacteria include, for example, those described in the work of Neidhardt et al. (Backmann B.J., 1996, Derivations and Genotypes of some mutant derivatives of Escherichia coli K-12, pp.2460-2488, Table 1, In F.D. Neidhardt (ed.), Escherichia coli and Salmonella Cellular and Molecular Biology/Second Edition, American Society for Microbiology Press, Washington, D.C.). Examples of the Escherichia bacteria include, for example, Escherichia coli. Specific examples of Escherichia coli include, for example, Escherichia coli K-12 strains such as W3110 strain (ATCC 27325) and MG1655 strain (ATCC 47076); Escherichia coli K5 strain (ATCC 23506); Escherichia coli B strains such as BL21 (DE3) strain; and derivative strains thereof.

The Enterobacter bacteria are not particularly limited, and examples include those classified into the genus Enterobacter according to the taxonomy known to those skilled in the field of microbiology. Examples of the Enterobacter bacterium include, for example, Enterobacter agglomerans and Enterobacter aerogenes. Specific examples of Enterobacter agglomerans include, for example, the Enterobacter agglomerans ATCC 12287 strain. Specific examples of Enterobacter aerogenes include, for example, the Enterobacter aerogenes ATCC 13048 strain, NBRC 12010 strain (Biotechnol. Bioeng., 2007, Mar. 27;98(2):340-348), and AJ110637 strain (FERM BP-10955). Examples of the Enterobacter bacteria also include, for example, the strains described in European Patent Application Laid-open (EP-A) No. 0952221. In addition, Enterobacter agglomerans also include some strains classified as Pantoea agglomerans.

The Pantoea bacteria are not particularly limited, and examples include those classified into the genus Pantoea according to the taxonomy known to those skilled in the field of microbiology. Examples of the Pantoea bacteria include, for example, Pantoea ananatis, Pantoea stewartii, Pantoea agglomerans, and Pantoea citrea. Specific examples of Pantoea ananatis include, for example, the Pantoea ananatis LMG20103 strain, AJ13355 strain (FERM BP-6614), AJ13356 strain (FERM BP-6615), AJ13601 strain (FERM BP-7207), SC17 strain (FERM BP-11091), SC17(0) strain (VKPM B-9246), and SC17sucA strain (FERM BP-8646). Some of Enterobacter bacteria and Erwinia bacteria were reclassified into the genus Pantoea (Int. J. Syst. Bacteriol., 39, 337-345 (1989); Int. J. Syst. Bacteriol., 43, 162-173 (1993)). For example, some strains of Enterobacter agglomerans were recently reclassified into Pantoea agglomerans, Pantoea ananatis, Pantoea stewartii, or the like on the basis of nucleotide sequence analysis of 16S rRNA etc. (Int. J. Syst. Bacteriol., 39, 337-345 (1989)). In the present invention, the Pantoea bacteria include those reclassified into the genus Pantoea as described above.

Examples of the Erwinia bacteria include Erwinia amylovora and Erwinia carotovora. Examples of the Klebsiella bacteria include Klebsiella planticola.

The bacteria belonging to the family Enterobacteriaceae have been recently reclassified into several families based on comprehensive comparative genome analysis (Adelou M. et al., Genome-based phylogeny and taxonomy of the `Enterobacteriales' : proposal for Enterobacterales ord. nov. divided into the families Enterobacteriaceae, Erwiniaceae fam. nov., Pectobacteriaceae fam. nov., Yersiniaceae fam. nov., Hafniaceae fam. nov., Morganellaceae fam. nov., and Budviciaceae fam. nov., Int. J. Syst. Evol. Microbiol., 2016, 66:5575-5599). However, herein, bacteria that had been conventionally classified into the family Enterobacteriaceae are dealt as bacteria belonging to the family Enterobacteriaceae. That is, the bacteria exemplified above such as the Pantoea bacteria are dealt as bacteria belonging to the family Enterobacteriaceae regardless of the current classification.

These strains are available from, for example, the American Type Culture Collection (Address: 12301 Parklawn Drive, Rockville, Maryland 20852, P.O. Box 1549, Manassas, VA 20108, United States of America). That is, registration numbers are given to the respective strains, and the strains can be ordered by using these registration numbers (refer to http://www.atcc.org/). The registration numbers of the strains are listed in the catalogue of the American Type Culture Collection. These strains can also be obtained from, for example, the depositories at which the strains were deposited.

The bacterium of the present invention may be a bacterium inherently having the L-amino acid-producing ability or may be a bacterium modified so that it has the L-amino acid-producing ability. The bacterium having the L-amino acid-producing ability can be obtained by imparting the L-amino acid-producing ability to such a bacterium as mentioned above, or by enhancing the L-amino acid-producing ability of such a bacterium as mentioned above.

To impart or enhance the L-amino acid-producing ability, methods conventionally employed in the breeding of amino acid-producing strains of coryneform bacteria, Escherichia bacteria, and so forth (refer to "Amino Acid Fermentation", Gakkai Shuppan Center (Ltd.), 1st Edition, published May 30, 1986, pp.77-100) can be used. Examples of such methods include, for example, acquiring an auxotrophic mutant strain, acquiring an L-amino acid analogue-resistant strain, acquiring a metabolic regulation mutant strain, and constructing a recombinant strain in which the activity of an L-amino acid biosynthetic enzyme is enhanced. In the breeding of L-amino acid-producing bacteria, one of the above-described properties such as auxotrophy, analogue resistance, and metabolic regulation mutation may be imparted alone, or two or three or more of such properties may be imparted in combination. Also, in the breeding of L-amino acid-producing bacteria, the activity of one of L-amino acid biosynthetic enzymes may be enhanced alone, or the activities of two or three or more of such enzymes may be enhanced in combination. Furthermore, imparting property(s) such as auxotrophy, analogue resistance, and metabolic regulation mutation can be combined with enhancing the activity(s) of biosynthetic enzyme(s).

An auxotrophic mutant strain, analogue-resistant strain, or metabolic regulation mutant strain having the L-amino acid-producing ability can be obtained by subjecting a parental strain or wild-type strain to a usual mutagenesis treatment, and then selecting a strain exhibiting autotrophy, analogue resistance, or a metabolic regulation mutation, and having the L-amino acid-producing ability from the obtained mutant strains. Examples of the usual mutagenesis treatment include irradiation of X-ray or ultraviolet and a treatment with a mutation agent such as N-methyl-N'-nitro-N-nitrosoguanidine (MNNG), ethyl methanesulfonate (EMS), and methyl methanesulfonate (MMS).

The L-amino acid-producing ability can also be imparted or enhanced by enhancing the activity of an enzyme involved in biosynthesis of an objective L-amino acid. An enzyme activity can be enhanced by, for example, modifying a bacterium so that the expression of a gene encoding the enzyme is enhanced. Methods for enhancing gene expression are described in WO00/18935, EP1010755A, and so forth. The detailed procedures for enhancing enzyme activity will be described below.

The L-amino acid-producing ability can also be imparted or enhanced by reducing the activity of an enzyme that catalyzes a reaction branching away from the biosynthetic pathway of an objective L-amino acid to generate a compound other than the objective L-amino acid. The "enzyme that catalyzes a reaction branching away from the biosynthetic pathway of an objective L-amino acid to generate a compound other than the objective L-amino acid" referred to herein includes an enzyme involved in decomposition of the objective amino acid. The method for reducing an enzyme activity will be described below.

Hereinafter, L-amino acid-producing bacteria and methods for imparting or enhancing the L-amino acid-producing ability will be specifically exemplified. All the properties of the L-amino acid-producing bacteria and modifications for imparting or enhancing the L-amino acid-producing ability may be used independently or in any appropriate combination.

### <L-Glutamic acid-producing bacteria>

Examples of methods for imparting or enhancing L-glutamic acid-producing ability include, for example, a method of modifying a bacterium so that the bacterium has an increased activity or activities of one or more kinds of enzymes selected from the L-glutamic acid biosynthesis enzymes. Examples of such enzymes include, but not particularly limited to, glutamate dehydrogenase (gdhA), glutamine synthetase (glnA), glutamate synthase (gltBD), isocitrate dehydrogenase (icdA), aconitate hydratase (acnA, acnB), citrate synthase (gltA), methylcitrate synthase (prpC), pyruvate carboxylase (pyc), pyruvate dehydrogenase (aceEF, lpdA), pyruvate kinase (pykA, pykF), phosphoenolpyruvate synthase (ppsA), enolase (eno), phosphoglyceromutase (pgmA, pgmI), phosphoglycerate kinase (pgk), glyceraldehyde-3-phophate dehydrogenase (gapA), triose phosphate isomerase (tpiA), fructose bisphosphate aldolase (fbp), glucose phosphate isomerase (pgi), 6-phosphogluconate dehydratase (edd), 2-keto-3-deoxy-6-phosphogluconate aldolase (eda), and transhydrogenase (pntAB). Shown in the parentheses after the names of the enzymes are examples of genes encoding the enzymes (the same shall apply to the same occasions hereinafter). It is preferable to enhance the activity or activities of one or more kinds of enzymes selected from, for example, glutamate dehydrogenase, citrate synthase, phosphoenol pyruvate carboxylase, and methylcitrate synthase, among these enzymes.

Examples of strains belonging to the family Enterobacteriaceae and modified so that the expression of the citrate synthase gene, phosphoenolpyruvate carboxylase gene, and/or glutamate dehydrogenase gene are increased include those disclosed in EP1078989A, EP955368A, and EP952221A. Furthermore, examples of strains belonging to the family Enterobacteriaceae and modified so that the expression of a gene of the Entner-Doudoroff pathway (edd, eda) is increased include those disclosed in EP1352966B.

Examples of methods for imparting or enhancing L-glutamic acid-producing ability also include, for example, a method of modifying a bacterium so that the bacterium has a reduced activity or activities of one or more kinds of enzymes selected from the enzymes that catalyze a reaction branching away from the biosynthesis pathway of L-glutamic acid to generate a compound other than L-glutamic acid. Examples of such enzymes include, but not particularly limited to, isocitrate lyase (aceA), α-ketoglutarate dehydrogenase (sucA), acetolactate synthase (ilvI), formate acetyltransferase (pfl), lactate dehydrogenase (ldh), alcohol dehydrogenase (adh), glutamate decarboxylase (gadAB), and succinate dehydrogenase (sdhABCD). It is preferable to reduce or delete, for example, the α-ketoglutarate dehydrogenase activity, among these enzymes.

Escherichia bacteria having a reduced α-ketoglutarate dehydrogenase activity or deficient in the α-ketoglutarate dehydrogenase activity, and methods for obtaining them are described in U.S. Patent Nos. 5,378,616 and 5,573,945. Furthermore, methods for reducing or deleting the α-ketoglutarate dehydrogenase activity of Enterobacteriaceae bacteria such as Pantoea bacteria, Enterobacter bacteria, Klebsiella bacteria, and Erwinia bacteria are disclosed in U.S. Patent Nos. 6,197,559, 6,682,912, 6,331,419, and 8,129,151, and WO2008/075483. Specific examples of Escherichia bacteria having a reduced α-ketoglutarate dehydrogenase activity or deficient in the α-ketoglutarate dehydrogenase activity include the following strains.
E. coli W3110sucA::Km^{r}
E. coli AJ12624 (FERM BP-3853)
E. coli AJ12628 (FERM BP-3854)
E. coli AJ12949 (FERM BP-4881)

E. coli W3110sucA::Km^{r} is a strain obtained by disrupting the sucA gene encoding α-ketoglutarate dehydrogenase of E. coli W3110. This strain is completely deficient in the α-ketoglutarate dehydrogenase activity.

Examples of L-glutamic acid-producing bacteria and parental strains for deriving them also include Pantoea bacteria, such as the Pantoea ananatis AJ13355 strain (FERM BP-6614), Pantoea ananatis SC17 strain (FERM BP-11091), and Pantoea ananatis SC17(0) strain (VKPM B-9246). The AJ13355 strain is a strain isolated from soil in Iwata-shi, Shizuoka-ken, Japan as a strain that can proliferate in a low pH culture medium containing L-glutamic acid and a carbon source. The SC17 strain is a strain selected as a low phlegm-producing mutant strain from the AJ13355 strain (U.S. Patent No. 6,596,517). The SC17 strain was deposited at the independent administrative agency, National Institute of Advanced Industrial Science and Technology, International Patent Organism Depository (currently independent administrative agency, National Institute of Technology and Evaluation, International Patent Organism Depositary, #120, 2-5-8 Kazusakamatari, Kisarazu-shi, Chiba-ken, 292-0818, Japan) on February 4, 2009, and assigned an accession number of FERM BP-11091. The AJ13355 strain was deposited at the National Institute of Bioscience and Human Technology, Agency of Industrial Science and Technology (currently, independent administrative agency, National Institute of Technology and Evaluation, International Patent Organism Depositary, #120, 2-5-8 Kazusakamatari, Kisarazu-shi, Chiba-ken, 292-0818, Japan) on February 19, 1998 and assigned an accession number of FERM P-16644. Then, the deposit was converted to an international deposit under the provisions of Budapest Treaty on January 11, 1999, and assigned an accession number of FERM BP-6614. The strain SC17(0) was deposited in the Russian National Collection of Industrial Microorganisms (VKPM; FGUP GosNII Genetika, Russian Federation, 117545 Moscow, 1st Dorozhny proezd, 1) on September 21, 2005 under the accession number of VKPM B-9246.

Furthermore, examples of L-glutamic acid-producing bacteria and parental strains for deriving them also include Pantoea bacteria having a reduced α-ketoglutarate dehydrogenase activity or deficient in the α-ketoglutarate dehydrogenase activity. Examples of such strains include the AJ13356 strain (U.S. Patent No. 6,331,419), which is an α-ketoglutarate dehydrogenase E1 subunit (sucA) gene-deficient strain of the AJ13355 strain, and the SC17sucA strain (U.S. Patent No. 6,596,517), which is a sucA gene-deficient strain of the SC17 strain. The AJ13356 strain was deposited at the National Institute of Bioscience and Human-Technology, Agency of Industrial Science and Technology (currently, independent administrative agency, National Institute of Technology and Evaluation, International Patent Organism Depositary, #120, 2-5-8 Kazusakamatari, Kisarazu-shi, Chiba-ken, 292-0818, Japan) on February 19, 1998, and assigned an accession number of FERM P-16645. Then, the deposit was converted into an international deposit under the provisions of the Budapest Treaty on January 11, 1999, and assigned an accession number of FERM BP-6616. The SC17sucA strain was assigned a private number of AJ417, and deposited at the independent administrative agency, National Institute of Advanced Industrial Science and Technology, International Patent Organism Depositary (currently, independent administrative agency, National Institute of Technology and Evaluation, International Patent Organism Depositary, #120, 2-5-8 Kazusakamatari, Kisarazu-shi, Chiba-ken, 292-0818, Japan) on February 26, 2004, under an accession number of FERM BP-8646.

The AJ13355 strain was identified as Enterobacter agglomerans when it was isolated, but it was recently reclassified as Pantoea ananatis on the basis of nucleotide sequencing of 16S rRNA and so forth. Therefore, although the AJ13355 and AJ13356 strains are deposited at the aforementioned depository as Enterobacter agglomerans, they are referred to as Pantoea ananatis in this specification.

Furthermore, examples of L-glutamic acid-producing bacteria and parent strains for deriving them also include Pantoea bacteria such as the Pantoea ananatis SC17sucA/RSFCPG+pSTVCB strain, Pantoea ananatis AJ13601 strain, Pantoea ananatis NP106 strain, and Pantoea ananatis NA1 strain. The SC17sucA/RSFCPG+pSTVCB strain is a strain obtained by introducing into the SC17sucA strain a plasmid RSFCPG, which contains a citrate synthase gene (gltA), phosphoenolpyruvate carboxylase gene (ppc), and glutamate dehydrogenase gene (gdhA) derived from Escherichia coli, and a plasmid pSTVCB, which contains a citrate synthase gene (gltA) derived from Brevibacterium lactofermentum. The AJ13601 strain is a strain selected from the SC17sucA/RSFCPG+pSTVCB strain as a strain resistant to a high concentration of L-glutamic acid at a low pH. The NP106 strain is a strain obtained from the AJ13601 strain by curing the plasmids RSFCPG and pSTVCB. The NA1 strain is a strain obtained by introducing a plasmid RSFPPG into the NP106 strain (WO2010/027045). The plasmid RSFPPG has a structure in which the gltA gene of the plasmid RSFCPG was replaced with a methylcitrate synthase gene (prpC), and that is, contains the prpC gene, ppc gene, and gdhA gene (WO2008/020654). The AJ13601 strain was deposited at the National Institute of Bioscience and Human Technology, Agency of Industrial Science and Technology (currently, independent administrative agency, National Institute of Technology and Evaluation, International Patent Organism Depositary, #120, 2-5-8 Kazusakamatari, Kisarazu-shi, Chiba-ken, 292-0818, Japan) on August 18, 1999, and assigned an accession number FERM P-17516. Then, the deposit was converted to an international deposit under the provisions of the Budapest Treaty on July 6, 2000, and assigned an accession number FERM BP-7207.

Examples of L-glutamic acid-producing bacteria and parent strains for deriving them also include strains in which both the α-ketoglutarate dehydrogenase (sucA) activity and the succinate dehydrogenase (sdh) activity are reduced or deleted (Japanese Patent Laid-open (Kokai) No. 2010-041920). Specific examples of such strains include, for example, a sucAsdhA double-deficient strain of the Pantoea ananatis NA1 strain (Japanese Patent Laid-open (Kokai) No. 2010-041920).

Examples of L-glutamic acid-producing bacteria and parental strains for deriving them also include auxotrophic mutant strains. Specific examples of auxotrophic mutant strains include, for example, E. coli VL334thrC⁺(VKPM B-8961, EP1172433). E. coli VL334 (VKPM B-1641) is an L-isoleucine and L-threonine auxotrophic strain having mutations in the thrC and ilvA genes (U.S. Patent No. 4,278,765). E. coli VL334thrC⁺ is an L-isoleucine-auxotrophic L-glutamic acid-producing bacterium obtained by introducing a wild-type allele of the thrC gene into the VL334 strain. The wild-type allele of the thrC gene was introduced by the method of general transduction using a bacteriophage P1 grown on the wild-type E. coli K-12 strain (VKPM B-7) cells.

Examples of L-glutamic acid-producing bacteria and parental strains for deriving them also include strains having resistance to an aspartic acid analogue. Such strains can also be deficient in the α-ketoglutarate dehydrogenase activity. Specific examples of strains having resistance to an aspartic acid analogue and deficient in the α-ketoglutarate dehydrogenase activity include, for example, E. coli AJ13199 (FERM BP-5807, U.S. Patent No. 5,908,768), E. coli FERM P-12379, which additionally has a lowered L-glutamic acid-decomposing ability (U.S. Patent No. 5,393,671), and E. coli AJ13138 (FERM BP-5565, U.S. Patent No. 6,110,714).

Examples of methods for imparting or enhancing L-glutamic acid-producing ability also include, for example, a method of enhancing the expression of an L-glutamic acid secretion gene, such as yhfK gene (WO2005/085419) or ybjL gene (WO2008/133161).

The methods for imparting or enhancing L-glutamic acid-producing ability can also be effective for imparting or enhancing an ability to produce L-amino acids that are biosynthesized via L-glutamic acid as an intermediate, such as L-glutamine, L-proline, L-arginine, L-citrulline, and L-ornithine. Hence, a bacterium having an ability to produce any of these L-amino acids that are biosynthesized via L-glutamic acid may have, as required, such a property possessed by an L-glutamic acid-producing bacterium as mentioned above. For example, a bacterium having an ability to produce any of these L-amino acids that are biosynthesized via L-glutamic acid may have been modified so that the activity of α-ketoglutarate dehydrogenase and/or succinate dehydrogenase is reduced.

### <L-Glutamine-producing bacteria>

Examples of the method for imparting or enhancing L-glutamine-producing ability include, for example, a method of modifying a bacterium so that the activity or activities of one or more kinds of enzymes selected from the L-glutamine biosynthesis enzymes are enhanced. Examples of such enzymes include, but not particularly limited to, glutamate dehydrogenase (gdhA) and glutamine synthetase (glnA). The glutamine synthetase activity can also be enhanced by disruption of the glutamine adenylyltransferase gene (glnE) or disruption of the PII control protein gene (glnB) (EP1229121).

Examples of the method for imparting or enhancing L-glutamine-producing ability also include, for example, a method of modifying a bacterium so that the activity or activities of one or more kinds of enzymes that catalyze a reaction branching away from the biosynthesis pathway of L-glutamine to generate a compound other than L-glutamine are reduced. Examples of such enzymes include, but not particularly limited to, glutaminase.

Specific examples of L-glutamine-producing bacteria and parent strains for deriving them include, for example, a strain belonging to the genus Escherichia and having a mutant glutamine synthetase in which the tyrosine residue of the position 397 of glutamine synthetase has been replaced with another amino acid residue (US2003-0148474A).

### <L-Proline-producing bacteria>

Examples of methods for imparting or enhancing L-proline-producing ability include, for example, a method of modifying a bacterium so that the bacterium has an increased activity or activities of one or more kinds of enzymes selected from the L-proline biosynthesis enzymes. Examples of such enzymes include glutamate-5-kinase (proB), γ-glutamylphosphate reductase, and pyroline-5-carboxylate reductase (putA). For enhancing the activity of such an enzyme, for example, the proB gene encoding a glutamate-5-kinase desensitized to feedback inhibition by L-proline (German Patent No. 3127361) can be preferably used.

Examples of methods for imparting or enhancing L-proline-producing ability also include, for example, a method of modifying a bacterium so that the bacterium has a reduced activity of an enzyme involved in decomposition of L-proline. Examples of such an enzyme include proline dehydrogenase and ornithine aminotransferase.

Specific examples of L-proline-producing bacteria and parental strains for deriving them include, for example, E. coli NRRL B-12403 and NRRL B-12404 (British Patent No. 2075056), E. coli VKPM B-8012 (Russian Patent Application No. 2000124295), E. coli plasmid mutant strains described in German Patent No. 3127361, E. coli plasmid mutant strains described by Bloom F.R. et al. (The 15th Miami winter symposium, 1983, p. 34), E. coli 702 strain (VKPM B-8011), which is a 3,4-dehydroxyproline and azetidine-2-carboxylate resistant strain, and E. coli 702ilvA strain (VKPM B-8012), which is an ilvA gene-deficient strain of the 702 strain (EP1172433).

### <L-Arginine-producing bacteria>

Examples of methods for imparting or enhancing L-arginine-producing ability include, for example, a method of modifying a bacterium so that the bacterium has an increased activity or activities of one or more kinds of enzymes selected from the L-arginine biosynthesis enzymes. Examples of such enzymes include, but not particularly limited to, N-acetylglutamate synthase (argA), N-acetylglutamate kinase (argB), N-acetylglutamyl phosphate reductase (argC), acetylornithine transaminase (argD), acetylornithine deacetylase (argE), ornithine carbamoyl transferase (argF, argI), argininosuccinate synthetase (argG), argininosuccinate lyase (argH), ornithine acetyl transferase (argJ), and carbamoyl phosphate synthetase (carAB). As the N-acetylglutamate synthase gene (argA), for example, a gene encoding a mutant N-acetylglutamate synthase desensitized to feedback inhibition by L-arginine by substitution for the amino acid residues corresponding to the positions 15 to 19 of the wild type enzyme (EP1170361A) can preferably be used.

Specific examples of L-arginine-producing bacteria and parental strains for deriving them include, for example, the E. coli 237 strain (VKPM B-7925, US2002-058315A1), derivative strains thereof introduced with the argA gene encoding a mutant N-acetyl glutamate synthase (Russian Patent Application No. 2001112869, EP1170361A1), E. coli 382 strain derived from the 237 strain and having an improved acetic acid-assimilating ability (VKPM B-7926, EP1170358A1), and E. coli 382ilvA+ strain, which is a strain obtained from the 382 strain by introducing the wild-type ilvA gene from E. coli K-12 strain thereto. The E. coli strain 237 was deposited at the Russian National Collection of Industrial Microorganisms (VKPM, FGUP GosNII Genetika, 1 Dorozhny proezd., 1 Moscow 117545, Russia) on April 10, 2000 under an accession number of VKPM B-7925, and the deposit was converted to an international deposit under the provisions of Budapest Treaty on May 18, 2001. The E. coli 382 strain was deposited at the Russian National Collection of Industrial Microorganisms (VKPM, FGUP GosNII Genetika, 1 Dorozhny proezd., 1 Moscow 117545, Russia) on April 10, 2000 under accession number of VKPM B-7926.

Examples of L-arginine-producing bacteria and parental strains for deriving them also include strains having resistance to amino acid analogues, and so forth. Examples of such strains include E. coli mutant strains having resistance to α-methylmethionine, p-fluorophenylalanine, D-arginine, arginine hydroxamate, S-(2-aminoethyl)-cysteine, α-methylserine, β-2-thienylalanine, or sulfaguanidine (Japanese Patent Laid-open (Kokai) No. 56-106598).

### <L-Citrulline-producing bacteria and L-ornithine-producing bacteria>

L-citrulline and L-omithine are intermediates of the biosynthetic pathway of L-arginine. Hence, examples of methods for imparting or enhancing an ability to produce L-citrulline and/or L-ornithine include, for example, a method of modifying a bacterium so that the bacterium has an increased activity or activities of one or more kinds of enzymes selected from the L-arginine biosynthesis enzymes. Examples of such enzymes include, but not particularly limited to, N-acetylglutamate synthase (argA), N-acetylglutamate kinase (argB), N-acetylglutamyl phosphate reductase (argC), acetyl ornithine transaminase (argD), acetyl ornithine deacetylase (argE), ornithine carbamoyl transferase (argF, argI), ornithine acetyl transferase (argJ), and carbamoyl phosphate synthetase (carAB), for L-citrulline. Furthermore, Examples of such enzymes include, but not particularly limited to, N-acetylglutamate synthase (argA), N-acetylglutamate kinase (argB), N-acetylglutamyl phosphate reductase (argC), acetyl ornithine transaminase (argD), acetyl ornithine deacetylase (argE), and ornithine acetyl transferase (argJ), for L-omithine.

An L-citrulline-producing bacterium can be easily obtained from, for example, an L-arginine bacterium such as the E. coli 382 strain (VKPM B-7926) by reducing the activity of argininosuccinate synthetase encoded by argG gene. Also, an L-ornithine-producing bacterium can be easily obtained from, for example, an L-arginine bacterium such as the E. coli 382 strain (VKPM B-7926) by reducing the activity of ornithine carbamoyl transferase encoded by argF and argI genes.

Specific examples of L-citrulline-producing bacteria and parental strains for deriving them include, for example, strains belonging to the genus Escherichia, such as the E. coli strains 237/pMADS11, 237/pMADS12, and 237/pMADS13, which have a mutant N-acetylglutamate synthase (Russian patent No. 2,215,783, U.S. Patent No. 6,790,647, and EP1170361B1), E. coli strains 333 (VKPM B-8084) and 374 (VKPM B-8086), which have carbamoyl phosphate synthetase resistant to feedback inhibition (Russian patent No. 2,264,459), and E. coli strains having an increased activity of α-ketoglutarate synthase and having a modified activity of ferredoxin NADP⁺ reductase, pyruvate synthase, and/or α-ketoglutarate dehydrogenase (EP2133417A). Specific examples of L-citrulline-producing bacteria and parental strains for deriving them also include, for example, strains belonging to the genus Pantoea, such as the P. ananatis NA1sucAsdhA strain, which has reduced activities of succinate dehydrogenase and α-ketoglutarate dehydrogenase (US2009-286290A1).

Furthermore, examples of methods for imparting or enhancing the L-amino acid-producing ability include, for example, a method of modifying a bacterium so that the bacterium has an increased activity for secreting an L-amino acid from a bacterial cell. Such an activity for secreting an L-amino acid can be increased by, for example, increasing the expression of a gene encoding a protein responsible for secretion of the L-amino acid. Examples of genes encoding the proteins responsible for secretion of various amino acids include, for example, b2682 gene (ygaZ), b2683 gene (ygaH), b1242 gene (ychE), and b3434 gene (yhgN) (Japanese Patent Laid-open (Kokai) No. 2002-300874).

Furthermore, examples of methods for imparting or enhancing the L-amino acid-producing ability include, for example, a method of modifying a bacterium so that the bacterium has an increased activity or activities of one or more kinds of proteins selected from proteins involved in the glycometabolism and proteins involved in the energy metabolism.

Examples of the proteins involved in the glycometabolism include proteins involved in uptake of saccharides and the glycolysis system enzymes. Examples of genes encoding a protein involved in the glycometabolism include glucose-6-phosphate isomerase gene (pgi, WO01/02542), pyruvate carboxylase gene (pyc, WO99/18228, EP1092776A), phosphoglucomutase gene (pgm, WO03/04598), fructose bisphosphate aldolase gene (pfkB, fbp, WO03/04664), transaldolase gene (talB, WO03/008611), fumarase gene (fum, WO01/02545), non-PTS sucrose uptake gene (csc, EP1149911A), and sucrose assimilation gene (scrAB operon, U.S. Patent No. 7,179,623).

Examples of genes encoding the proteins involved in the energy metabolism include the transhydrogenase gene (pntAB, U.S. Patent No. 5,830,716) and cytochrome bo-type oxidase gene (cyoB, EP1070376A).

Furthermore, examples of methods for imparting or enhancing an ability to produce useful substances such as L-amino acids include, for example, a method of modifying a bacterium so that the activity of phosphoketolase is increased (WO2006/016705). Hence, the bacterium of the present invention may have been modified so that the activity of phosphoketolase is increased. The increase in the activity of phosphoketolase may be effective particularly for imparting or enhancing an ability to produce an L-amino acid of glutamate family such as L-glutamic acid. Examples of phosphoketolase include D-xylulose-5-phosphate phosphoketolase and fructose-6-phosphate phosphoketolase. Either one of the D-xylulose-5-phosphate phosphoketolase activity and the fructose-6-phosphate phosphoketolase activity may be enhanced, or both may be enhanced.

The phrase "D-xylulose-5-phosphate phosphoketolase activity" refers to an activity for converting xylulose-5-phosphate into glycelaldehyde-3-phosphate and acetyl phosphate with consuming phosphoric acid to release one molecule of H₂O. This activity can be measured by the method described by Goldberg, M. et al. (Methods Enzymol., 9, 515-520, 1966) or the method described by L. Meile (J. Bacteriol., 183:2929-2936, 2001). Examples of D-xylulose-5-phosphate phosphoketolase include those of bacteria belonging to the genera Acetobacter, Bifidobacterium, Lactobacillus, Thiobacillus, Streptococcus, Methylococcus, Butyrivibrio, and Fibrobacter, and yeast belonging to the genera Candida, Rhodotorula, Rhodosporidium, Pichia, Yarrowia, Hansenula, Kluyveromyces, Saccharomyces, Trichosporon, and Wingea. Specific examples of D-xylulose-5-phosphate phosphoketolase and genes encoding them are disclosed in WO2006/016705.

The phrase "fructose-6-phosphate phosphoketolase activity" refers to an activity for converting fructose-6-phosphate into erythrose-4-phosphate and acetyl phosphate with consuming phosphoric acid to release one molecule of H₂O. This activity can be measured by the method described by Racker, E. (Methods Enzymol., 5, 276-280, 1962) or the method described by L. Meile (J. Bacteriol., 183:2929-2936, 2001). Examples of fructose-6-phosphate phosphoketolase include those of bacteria belonging to the genera Acetobacter, Bifidobacterium, Chlorobium, Brucella, Methylococcus, and Gardnerella, and yeast belonging to the genera Rhodotorula, Candida, and Saccharomyces. Specific examples of fructose-6-phosphate phosphoketolase and genes encoding them are disclosed in WO2006/016705.

Both phosphoketolase activities (namely, the D-xylulose-5-phosphate phosphoketolase activity and the fructose-6-phosphate phosphoketolase activity) may also be retained by a single enzyme (namely, D-xylulose-5-phosphate phosphoketolase/fructose-6-phosphate phosphoketolase).

The genes and proteins to be used for breeding L-amino acid-producing bacteria may have, for example, the nucleotide sequences and amino acid sequences of known genes and proteins, such as those exemplified above, respectively. Also, the genes and proteins to be used for breeding L-amino acid-producing bacteria may be conservative variants of known genes and proteins, such as those exemplified above, respectively. Specifically, for example, the genes to be used for breeding L-amino acid-producing bacteria may each be a gene encoding a protein having an amino acid sequence of a known protein, but including substitution, deletion, insertion, or addition of one or several amino acid residues at one or several positions, so long as the original function thereof is maintained. The genes used for breeding L-amino acid-producing bacteria may each have been modified to have optimal codons depending on the codon usage of the host to be used. For the conservative variants of genes and proteins, the descriptions concerning conservative variants of the target genes and the target proteins mentioned later can be similarly applied.

### <1-2> Specific feature

The bacterium of the present invention has been modified to have the specific feature. The bacterium of the present invention can be obtained by modifying a bacterium having the L-amino acid-producing ability to have the specific feature. The bacterium of the present invention can also be obtained by modifying a bacterium to have the specific feature, and then imparting or enhancing the L-amino acid-producing ability. The bacterium of the present invention may also be a bacterium that has acquired the L-amino acid-producing ability by being modified to have the specific feature. The bacterium of the present invention may have, as required, such a property possessed by an L-amino acid-producing bacterium as mentioned above, as well as being modified to have the specific feature. The modifications for constructing the bacterium of the present invention can be performed in any order.

By modifying a bacterium to have the specific feature, the L-amino acid-producing ability of the bacterium can be improved, and that is, production of the L-amino acid using the bacterium can be increased. Examples of the increase in production of the L-amino acid include an increase in the accumulation amount of the L-amino acid and an increase in the yield of the L-amino acid.

Examples of the specific feature include the modifications (A) to (F) shown below:
(A) modification of reducing the activity of a BudA protein;
(B) modification of reducing the activity of a BudB protein;
(C) modification of reducing the activity of a BudC protein;
(D) modification of reducing the activity of a PAJ_3461 protein;
(E) modification of reducing the activity of a PAJ 3462 protein; and
(F) modification of reducing the activity of a PAJ_3463 protein.

The bacterium of the present invention may have, for example, one or more modifications, for example, 1, 2, 3, 4, 5, or all the 6 modifications, selected from the modifications (A) to (F).

The bacterium of the present invention may at least have, for example, one or more modifications, for example, 1, 2, or all the 3 modifications, selected from the modifications (A) to (C). The bacterium of the present invention may have, for example, one or more modifications, for example, 1, 2, or all the 3 modifications, selected from the modifications (A) to (C), and may further have one or more modifications, for example, 1, 2, or all the 3 modifications, selected from the modifications (D) to (F).

The bacterium of the present invention may at least have, for example, one or more modifications, for example, 1, 2, or all the 3 modifications, selected from the modifications (D) to (F). The bacterium of the present invention may have, for example, one or more modifications, for example, 1, 2, or all the 3 modifications, selected from the modifications (D) to (F), and may further have one or more modifications, for example, 1, 2, or all the 3 modifications, selected from the modifications (A) to (C).

In an embodiment, it is acceptable that the modification (B) is not solely selected. That is, in an embodiment, the bacterium of the present invention may at least have one or more modifications, for example, 1, 2, 3, 4, or all the 5 modifications, selected from the modifications (A) and (C) to (F). In an embodiment, the bacterium of the present invention may have one or more modifications, for example, 1, 2, 3, 4, or all the 5 modifications, selected from the modifications (A) and (C) to (F), and may further have the modification (B).

By modifying a bacterium to have one or more modifications selected from the modifications (A) to (C) and one or more modifications selected from the modifications (D) to (F), the L-amino acid-producing ability of the bacterium may be further improved as compared to when the bacterium only has one or more modifications selected from the modifications (A) to (C) and/or when the bacterium only has one or more modifications selected from the modifications (D) to (F). For example, by modifying a bacterium to have the modifications (A) to (F), the L-amino acid-producing ability of the bacterium may be further improved as compared to when the bacterium only has the modifications (A) to (C) and/or when the bacterium only has the modifications (D) to (F).

The phrase "BudA protein" means a protein encoded by a budA gene. The BudA protein may be acetolactate decarboxylase. That is, the phrase "reduction in the activity of a BudA protein" may mean a reduction in acetolactate decarboxylase activity. The phrase "acetolactate decarboxylase" may mean a protein that catalyzes a reaction of decarboxylating acetolactic acid to generate acetoin and/or a reverse reaction thereof (EC 4.1.1.5 etc.). This activity is also referred to as "acetolactate decarboxylase activity". The acetolactate decarboxylase activity may be, particularly, an activity of catalyzing the following chemical reaction in either one direction or both directions.

(2S)-2-hydroxy-2-methyl-3-oxobutanoate = (3R)-3-hydroxybutan-2-one + CO₂

The nucleotide sequences of budA genes possessed by bacteria to be modified and the amino acid sequences of BudA proteins encoded thereby can be obtained from, for example, public databases such as NCBI. The budA gene (PAJ_p0041) of Pantoea ananatis AJ13355 corresponds to the complementary sequence of the nucleotide sequence at positions 44299 to 45081 in the nucleotide sequence of pEA320 plasmid of Pantoea ananatis AJ13355 registered as GenBank accession AP012033.1. The nucleotide sequence of the budA gene (PAJ_p0041) of Pantoea ananatis AJ13355 and the amino acid sequence of the BudA protein encoded by the gene are shown in SEQ ID NOS: 1 and 2, respectively.

The phrase "BudB protein" means a protein encoded by a budB gene. The BudB protein may be acetolactate synthase. That is, the phrase "reduction in the activity of a BudB protein" may mean a reduction in acetolactate synthase activity. The phrase "acetolactate synthase" may mean a protein that catalyzes a reaction of generating acetolactic acid from pyruvic acid and/or a reverse reaction thereof (EC 2.2.1.6 etc.). This activity is also referred to as "acetolactate synthase activity". The acetolactate synthase activity may be, specifically, an activity of catalyzing a reaction of generating one molecule of acetolactic acid and one molecule of carbon dioxide from 2 molecules of pyruvic acid and/or a reverse reaction thereof. The acetolactate synthase activity may be, particularly, an activity of catalyzing the following chemical reaction in either one direction or both directions.

2 pyruvate = 2-acetolactate + CO₂

The nucleotide sequences of budB genes possessed by bacteria to be modified and the amino acid sequences of BudB proteins encoded thereby can be obtained from, for example, public databases such as NCBI. The budB gene (PAJ_p0040) of Pantoea ananatis AJ13355 corresponds to the complementary sequence of the nucleotide sequence at positions 42603 to 44282 in the nucleotide sequence of pEA320 plasmid of Pantoea ananatis AJ13355 registered as GenBank accession AP012033.1. The nucleotide sequence of the budB gene (PAJ_p0040) of Pantoea ananatis AJ13355 and the amino acid sequence of the BudB protein encoded by the gene are shown in SEQ ID NOS: 3 and 4, respectively.

The phrase "BudC protein" means a protein encoded by a budC gene. The BudC protein may be (S,S)-butanediol dehydrogenase, meso-2,3-butandiol dehydrogenase, and/or diacetyl reductase. That is, the phrase "reduction in the activity of a BudC protein" may mean a reduction in (S,S)-butanediol dehydrogenase activity, meso-2,3-butandiol dehydrogenase activity, and/or diacetyl reductase activity.

The phrase "(S,S)-butanediol dehydrogenase" may mean a protein that catalyzes a reaction of oxidizing (S,S)-butanediol to generate (S)-acetoin and/or a reverse reaction thereof (EC 1.1.1.76 etc.). This activity is also referred to as "(S,S)-butanediol dehydrogenase activity". The (S,S)-butanediol dehydrogenase activity may be, specifically, an activity of catalyzing a reaction of oxidizing (S,S)-butanediol to generate (S)-acetoin in the presence of an electron acceptor and/or a reverse reaction thereof. Examples of the electron acceptor include NAD⁺. An electron donor may be used in the reverse reaction. Examples of the electron donor include NADH. The (S,S)-butanediol dehydrogenase activity may be, particularly, an activity of catalyzing the following chemical reaction in either one direction or both directions.

(2S,3S)-butane-2,3-diol + NAD⁺ = (S)-acetoin + NADH + H⁺

The phrase "meso-2,3-butandiol dehydrogenase" may mean a protein that catalyzes a reaction of oxidizing meso-2,3-butandiol to generate acetoin and/or a reverse reaction thereof (EC 1.1.1.B20 etc.). This activity is also referred to as "meso-2,3-butandiol dehydrogenase activity". The meso-2,3-butandiol dehydrogenase activity may be, specifically, an activity of catalyzing a reaction of oxidizing meso-2,3-butandiol to generate acetoin in the presence of an electron acceptor and/or a reverse reaction thereof. Examples of the electron acceptor include NAD⁺. An electron donor may be used in the reverse reaction. Examples of the electron donor include NADH. The meso-2,3-butandiol dehydrogenase activity may be, particularly, an activity of catalyzing the following chemical reaction in either one direction or both directions.

(2R,3S)-butane-2,3-diol + NAD⁺ = acetoin + NADH + H⁺

The phrase "diacetyl reductase" may mean a protein that catalyzes a reaction of oxidizing (S)-acetoin to generate diacetyl and/or a reverse reaction thereof (EC 1.1.1.304 etc.). This activity is also referred to as "diacetyl reductase activity". The diacetyl reductase activity may be, specifically, an activity of catalyzing a reaction of oxidizing (S)-acetoin to generate diacetyl in the presence of an electron acceptor and/or a reverse reaction thereof. Examples of the electron acceptor include NAD⁺. An electron donor may be used in the reverse reaction. Examples of the electron donor include NADH. The diacetyl reductase activity may be, particularly, an activity of catalyzing the following chemical reaction in either one direction or both directions.

(S)-acetoin + NAD⁺ = diacetyl + NADH + H⁺

The nucleotide sequences of budC genes possessed by bacteria to be modified and the amino acid sequences of BudC proteins encoded thereby can be obtained from, for example, public databases such as NCBI. The budC gene (PAJ_p0039) of Pantoea ananatis AJ13355 corresponds to the complementary sequence of the nucleotide sequence at positions 41805 to 42572 in the nucleotide sequence of pEA320 plasmid of Pantoea ananatis AJ13355 registered as GenBank accession AP012033.1. The nucleotide sequence of the budC gene (PAJ_p0039) of Pantoea ananatis AJ13355 and the amino acid sequence of the BudC protein encoded by the gene are shown in SEQ ID NOS: 5 and 6, respectively.

The phrases "PAJ_3461 protein", "PAJ_3462 protein", and "PAJ_3463 protein" mean proteins encoded by a PAJ_3461 gene, a PAJ_3462 gene, and a PAJ_3463 gene, respectively. The PAJ_3461 gene, PAJ_3462 gene, and PAJ_3463 gene are also collectively referred to as "GlcNDH genes". The PAJ_3461 protein, PAJ_3462 protein, and PAJ_3463 protein may each be gluconate 2-dehydrogenase. That is, the phrases "reduction in the activity of a PAJ_3461 protein", "reduction in the activity of a PAJ_3462 protein", and "reduction in the activity of a PAJ_3463 protein" may each mean a reduction in gluconate 2-dehydrogenase activity. The PAJ_3461 protein, PAJ_3462 protein, and PAJ_3463 protein may be, specifically, respective subunits constituting gluconate 2-dehydrogenase. The PAJ_3461 may be, more specifically, a subunit constituting gluconate 2-dehydrogenase, wherein the function of the subunit is unknown. The PAJ_3462 may be, more specifically, a dehydrogenase subunit constituting gluconate 2-dehydrogenase. The PAJ_3463 may be, more specifically, a cytochrome c subunit constituting gluconate 2-dehydrogenase. The PAJ_3461 protein, PAJ_3462 protein, and PAJ_3463 protein may, for example, form a complex consisting of those proteins, to function as gluconate 2-dehydrogenase. The phrase "gluconate 2-dehydrogenase" may mean a protein that catalyzes a reaction of oxidizing gluconic acid to generate 2-dehydro-gluconic acid and/or a reverse reaction thereof (EC 1.1.99.3 etc.). This activity is also referred to as "gluconate 2-dehydrogenase activity". The gluconate 2-dehydrogenase activity may be, specifically, an activity of catalyzing a reaction of oxidizing gluconic acid to generate 2-dehydro-gluconic acid in the presence of an electron acceptor and/or a reverse reaction thereof. Examples of the electron acceptor include oxidized quinones, NAD⁺, NADP⁺, and FAD⁺. An electron donor may be used in the reverse reaction. Examples of the electron donor include reduced quinones, NADH, NADPH, and FADH₂. The gluconate 2-dehydrogenase activity may be, particularly, an activity of catalyzing the following chemical reaction in either one direction or both directions.

D-gluconate + electron acceptor = 2-dehydro-D-gluconate + reduced electron acceptor

The phrase "a protein has gluconate 2-dehydrogenase activity" is not limited to when the protein independently has the gluconate 2-dehydrogenase activity and includes when the protein in combination with another subunit has the gluconate 2-dehydrogenase activity. The phrase "a protein independently has gluconate 2-dehydrogenase activity" may mean that the protein independently functions as gluconate 2-dehydrogenase. The phrase "a protein in combination with another subunit has gluconate 2-dehydrogenase activity" may mean that the protein in combination with another subunit functions as gluconate 2-dehydrogenase (for example, the protein forms a complex with another subunit, to function as gluconate 2-dehydrogenase.

Examples of the other subunit for the PAJ_3461 protein include the PAJ_3462 protein and PAJ_3463 protein. That is, the phrase "a protein has gluconate 2-dehydrogenase activity" for the PAJ_3461 protein is not limited to when the protein independently has the gluconate 2-dehydrogenase activity and includes when the protein in combination with the PAJ_3462 protein and/or PAJ_3463 protein, particularly in combination with the PAJ_3462 protein and PAJ_3463 protein, has the gluconate 2-dehydrogenase activity.

Examples of the other subunit for the PAJ_3462 protein include the PAJ_3461 protein and PAJ_3463 protein. That is, the phrase "a protein has gluconate 2-dehydrogenase activity" for the PAJ_3462 protein is not limited to when the protein independently has the gluconate 2-dehydrogenase activity and includes when the protein in combination with the PAJ_3461 protein and/or PAJ_3463 protein, particularly in combination with the PAJ_3461 protein and PAJ_3463 protein, has the gluconate 2-dehydrogenase activity.

Examples of the other subunit for the PAJ_3463 protein include the PAJ_3461 protein and PAJ_3462 protein. That is, the phrase "a protein has gluconate 2-dehydrogenase activity" for the PAJ_3463 protein is not limited to when the protein independently has the gluconate 2-dehydrogenase activity and includes when the protein in combination with the PAJ_3461 protein and/or PAJ_3462 protein, particularly in combination with the PAJ_3461 protein and PAJ_3462 protein, has the gluconate 2-dehydrogenase activity.

The nucleotide sequences of PAJ_3461 genes, PAJ_3462 genes, and PAJ_3463 genes possessed by bacteria to be modified and the amino acid sequences of PAJ_3461 proteins, PAJ_3462 proteins, and PAJ_3463 proteins encoded thereby can be obtained from, for example, public databases such as NCBI. The PAJ_3461 gene of Pantoea ananatis AJ13355 corresponds to the nucleotide sequence at positions 4149024 to 4149749 in the genome sequence of Pantoea ananatis AJ13355 registered as GenBank accession AP012032.2. The PAJ_3462 gene of Pantoea ananatis AJ13355 corresponds to the nucleotide sequence at positions 4149752 to 4151536 in the genome sequence of Pantoea ananatis AJ13355 registered as GenBank accession AP012032.2. The PAJ_3463 gene of Pantoea ananatis AJ13355 corresponds to the nucleotide sequence at positions 4151542 to 4152858 in the genome sequence of Pantoea ananatis AJ13355 registered as GenBank accession AP012032.2. The nucleotide sequence of the PAJ_3461 gene of Pantoea ananatis AJ13355 and the amino acid sequence of the PAJ_3461 protein encoded by the gene are shown in SEQ ID NOS: 7 and 8, respectively. The nucleotide sequence of the PAJ_3462 gene of Pantoea ananatis AJ13355 and the amino acid sequence of the PAJ_3462 protein encoded by the gene are shown in SEQ ID NOS: 9 and 10, respectively. The nucleotide sequence of the PAJ_3463 gene of Pantoea ananatis AJ13355 and the amino acid sequence of the PAJ_3463 protein encoded by the gene are shown in SEQ ID NOS: 11 and 12, respectively.

Regarding the modifications (A) to (F), the activity of each recited protein is reduced as compared with a non-modified strain. Methods for reducing the activity of a protein are described below. The activity of a protein can be reduced by, for example, reducing the expression of a gene encoding the protein or disrupting the gene. Such methods for reducing the activity of a protein can be used independently or in any appropriate combination.

The BudA protein, BudB protein, BudC protein, PAJ_3461 protein, PAJ_3462 protein, and PAJ_3463 protein are also collectively referred to as "target proteins". The budAgene, budB gene, budC gene, PAJ_3461 gene, PAJ_3462 gene, and PAJ_3463 gene are also collectively referred to as "target genes".

The target genes may each be, for example, a gene having the nucleotide sequence of any of the target genes exemplified above, such as the nucleotide sequence shown as SEQ ID NO: 1, 3, 5, 7, 9, or 11. Also, the target proteins may each be, for example, a protein having the amino acid sequence of any of the target proteins exemplified above, such as the amino acid sequence shown as SEQ ID NO: 2, 4, 6, 8, 10, or 12. The phrase "a gene or protein has a nucleotide or amino acid sequence" means that the gene or protein comprises the nucleotide or amino acid sequence unless otherwise stated, and includes when the gene or protein consists of the nucleotide or amino acid sequence.

The target genes may each be a variant of any of the target genes exemplified above (for example, a gene having the nucleotide sequence shown as SEQ ID NO: 1, 3, 5, 7, 9, or 11), so long as the original function thereof is maintained. Similarly, the target proteins may each be a variant of any of the target proteins exemplified above (for example, a protein having the amino acid sequence shown as SEQ ID NO: 2, 4, 6, 8, 10, or 12), so long as the original function thereof is maintained. Such a variant that maintains the original function thereof is also referred to as "conservative variant". The phrases "budAgene", "budB gene", "budC gene", "PAJ_3461 gene", "PAJ_3462 gene", and "PAJ_3463 gene" include not only the budAgene, budB gene, budC gene, PAJ_3461 gene, PAJ_3462 gene, and PAJ_3463 gene exemplified above, respectively, but also include conservative variants thereof. Similarly, the phrases "BudA protein", "BudB protein", "BudC protein", "PAJ_3461 protein", "PAJ_3462 protein", and "PAJ_3463 protein" include not only the BudA protein, BudB protein, BudC protein, PAJ_3461 protein, PAJ_3462 protein, and PAJ_3463 protein exemplified above, respectively, but also include conservative variants thereof. Examples of the conservative variants include, for example, homologues and artificially modified versions of the target genes and the target proteins exemplified above. Incidentally, the target protein whose activity is to be reduced is a target protein possessed by the bacterium to be modified, and in other words, a target protein encoded by a target gene possessed by the bacterium to be modified. The bacterium to be modified may have the target gene on a chromosome or on a structure outside the chromosome, such as a plasmid. The term "chromosome" may be used interchangeably to the term "genome".

The phrase "the original function is maintained" means that a variant of a gene or protein has a function (such as activity or property) corresponding to the function (such as activity or property) of the original gene or protein. The phrase "the original function is maintained" used for a gene means that a variant of the gene encodes a protein that maintains the original function. That is, the phrase "the original function is maintained" used for each target gene may mean that a variant of the gene encodes a protein having the corresponding activity of each target protein, which activity may be acetolactate decarboxylase activity for the BudA protein; acetolactate synthase activity for the BudB protein; (S,S)-butanediol dehydrogenase activity, meso-2,3-butandiol dehydrogenase activity, and/or diacetyl reductase activity for the BudC protein; and gluconate 2-dehydrogenase activity for the PAJ_3461 protein, PAJ_3462 protein, and PAJ_3463 protein. Also, the phrase "the original function is maintained" used for each target protein may mean that a variant of the protein has the corresponding activity of each target protein, which activity may be acetolactate decarboxylase activity for the BudA protein; acetolactate synthase activity for the BudB protein; (S,S)-butanediol dehydrogenase activity, meso-2,3-butandiol dehydrogenase activity, and/or diacetyl reductase activity for the BudC protein; and gluconate 2-dehydrogenase activity for the PAJ_3461 protein, PAJ_3462 protein, and PAJ_3463 protein.

The acetolactate decarboxylase activity can be measured by, for example, incubating the enzyme with a corresponding substrate, such as acetolactic acid, and measuring enzyme- and substrate-dependent generation of a corresponding product, such as acetoin. Also, in case of the reverse reaction, the acetolactate decarboxylase activity can be measured by, for example, incubating the enzyme with a corresponding substrate, such as acetoin and carbon dioxide, and measuring enzyme- and substrate-dependent generation of a corresponding product, such as acetolactic acid.

The acetolactate synthase activity can be measured by, for example, incubating the enzyme with a corresponding substrate, such as pyruvic acid, and measuring enzyme- and substrate-dependent generation of a corresponding product, such as acetolactic acid. Also, in case of the reverse reaction, the acetolactate synthase activity can be measured by, for example, incubating the enzyme with a corresponding substrate, such as acetolactic acid and carbon dioxide, and measuring enzyme- and substrate-dependent generation of a corresponding product, such as pyruvic acid.

The (S,S)-butanediol dehydrogenase activity can be measured by, for example, incubating the enzyme with a corresponding substrate, such as (S,S)-butanediol, in the presence of an electron acceptor, and measuring enzyme- and substrate-dependent generation of a corresponding product, such as (S)-acetoin. Also, in case of the reverse reaction, the (S,S)-butanediol dehydrogenase activity can be measured by, for example, incubating the enzyme with a corresponding substrate, such as (S)-acetoin, in the presence of an electron donor, and measuring enzyme- and substrate-dependent generation of a corresponding product, such as (S,S)-butanediol.

The meso-2,3-butandiol dehydrogenase activity can be measured by, for example, incubating the enzyme with a corresponding substrate, such as meso-2,3-butandiol, in the presence of an electron acceptor, and measuring enzyme- and substrate-dependent generation of a corresponding product, such as acetoin. Also, in case of the reverse reaction, the meso-2,3-butandiol dehydrogenase activity can be measured by, for example, incubating the enzyme with a corresponding substrate, such as acetoin, in the presence of an electron donor, and measuring enzyme- and substrate-dependent generation of a corresponding product, such as meso-2,3-butandiol.

The diacetyl reductase activity can be measured by, for example, incubating the enzyme with a corresponding substrate, such as (S)-acetoin, in the presence of an electron acceptor, and measuring enzyme- and substrate-dependent generation of a corresponding product, such as diacetyl. Also, in case of the reverse reaction, the diacetyl reductase activity can be measured by, for example, incubating the enzyme with a corresponding substrate, such as diacetyl, in the presence of an electron donor, and measuring enzyme- and substrate-dependent generation of a corresponding product, such as (S)-acetoin.

The gluconate 2-dehydrogenase activity can be measured by, for example, incubating the enzyme with a corresponding substrate, such as gluconic acid, in the presence of an electron acceptor, and measuring enzyme- and substrate-dependent generation of a corresponding product, such as 2-dehydro-gluconic acid. Also, in case of the reverse reaction, the gluconate 2-dehydrogenase activity can be measured by, for example, incubating the enzyme with a corresponding substrate, such as 2-dehydro-gluconic acid, in the presence of an electron donor, and measuring enzyme- and substrate-dependent generation of a corresponding product, such as gluconic acid.

Hereinafter, examples of the conservative variants will be explained.

Homologues of the target genes or homologues of the target proteins can be easily obtained from public databases by, for example, BLAST search or FASTA search using any of the nucleotide sequences of the target genes exemplified above or any of the amino acid sequences of the target proteins exemplified above as a query sequence. Furthermore, homologues of the target genes can be obtained by, for example, PCR using a chromosome of various organisms as the template, and oligonucleotides prepared on the basis of any of the nucleotide sequences of these known target genes as primers.

The target genes may each be a gene encoding a protein having any of the aforementioned amino acid sequences (for example, the amino acid sequence shown as SEQ ID NO: 2, 4, 6, 8, 10, or 12), but which includes substitution, deletion, insertion, and/or addition of one or several amino acid residues at one or several positions, so long as the original function is maintained. For example, the N-terminus and/or the C-terminus of the encoded protein may be elongated or shortened. Although the number meant by the phrase "one or several" mentioned above may differ depending on the positions of amino acid residues in the three-dimensional structure of the protein or the types of amino acid residues, specifically, it is, for example, 1 to 50, 1 to 40, or 1 to 30, preferably 1 to 20, more preferably 1 to 10, still more preferably 1 to 5, particularly preferably 1 to 3.

The aforementioned substitution, deletion, insertion, and/or addition of one or several amino acid residues are/is a conservative mutation that maintains the normal function of the protein. Typical examples of the conservative mutation are conservative substitutions. The conservative substitution is a mutation wherein substitution takes place mutually among Phe, Trp, and Tyr, if the substitution site is an aromatic amino acid; among Leu, Ile, and Val, if it is a hydrophobic amino acid; between Gln and Asn, if it is a polar amino acid; among Lys, Arg, and His, if it is a basic amino acid; between Asp and Glu, if it is an acidic amino acid; and between Ser and Thr, if it is an amino acid having a hydroxyl group. Examples of substitutions considered as conservative substitutions include, specifically, substitution of Ser or Thr for Ala, substitution of Gln, His, or Lys for Arg, substitution of Glu, Gln, Lys, His, or Asp for Asn, substitution of Asn, Glu, or Gln for Asp, substitution of Ser or Ala for Cys, substitution of Asn, Glu, Lys, His, Asp, or Arg for Gln, substitution of Gly, Asn, Gln, Lys, or Asp for Glu, substitution of Pro for Gly, substitution of Asn, Lys, Gln, Arg, or Tyr for His, substitution of Leu, Met, Val, or Phe for Ile, substitution of Ile, Met, Val, or Phe for Leu, substitution of Asn, Glu, Gln, His, or Arg for Lys, substitution of Ile, Leu, Val, or Phe for Met, substitution of Trp, Tyr, Met, Ile, or Leu for Phe, substitution of Thr or Ala for Ser, substitution of Ser or Ala for Thr, substitution of Phe or Tyr for Trp, substitution of His, Phe, or Trp for Tyr, and substitution of Met, Ile, or Leu for Val. Furthermore, such substitution, deletion, insertion, or addition of amino acid residues as mentioned above includes a naturally occurring mutation due to an individual difference, or a difference of species of the organism from which the gene is derived (mutant or variant).

The target genes may each be a gene encoding a protein having an amino acid sequence showing an identity of, for example, 50% or more, 65% or more, or 80% or more, preferably 90% or more, more preferably 95% or more, still more preferably 97% or more, particularly preferably 99% or more, to the total amino acid sequence of any of the aforementioned amino acid sequences, so long as the original function is maintained.

The target genes may also each be a gene, such as DNA, that is able to hybridize under stringent conditions with a probe that can be prepared from any of the aforementioned nucleotide sequences (for example, the nucleotide sequence shown as SEQ ID NO: 1, 3, 5, 7, 9, or 11), such as a sequence complementary to a partial or entire sequence of any of the aforementioned nucleotide sequences, so long as the original function is maintained. The phrase "stringent conditions" refers to conditions under which a so-called specific hybrid is formed, and a non-specific hybrid is not formed. Examples of the stringent conditions include those under which highly identical DNAs hybridize to each other, for example, DNAs not less than 50%, 65%, or 80% identical, preferably not less than 90% identical, more preferably not less than 95% identical, still more preferably not less than 97% identical, particularly preferably not less than 99% identical, hybridize to each other, and DNAs less identical than the above do not hybridize to each other, or conditions of washing of typical Southern hybridization, namely, conditions of washing once, preferably 2 or 3 times, at a salt concentration and temperature corresponding to 1 x SSC, 0.1% SDS at 60°C, preferably 0.1 x SSC, 0.1% SDS at 60°C, more preferably 0.1 x SSC, 0.1% SDS at 68°C.

The probe used for the aforementioned hybridization may be a part of a sequence that is complementary to the gene as described above. Such a probe can be prepared by PCR using oligonucleotides prepared on the basis of a known gene sequence as primers and a DNA fragment containing any of the aforementioned genes as a template. As the probe, for example, a DNA fragment having a length of about 300 bp can be used. When a DNA fragment having a length of about 300 bp is used as the probe, the washing conditions of the hybridization may be, for example, 50°C, 2 x SSC and 0.1% SDS.

Furthermore, since the degeneracy of codons differs depending on the host, any codons in the target genes may be replaced with respective equivalent codons. That is, the target genes may each be a variant of any of the target genes exemplified above due to the degeneracy of the genetic code.

The term "identity" between amino acid sequences means an identity calculated by blastp with default scoring parameters (namely, Matrix, BLOSUM62; Gap Costs, Existence = 11, Extension = 1; Compositional Adjustments, Conditional compositional score matrix adjustment). The term "identity" between nucleotide sequences means an identity calculated by blastn with default scoring parameters (namely, Match/Mismatch Scores = 1, -2; Gap Costs = Linear).

The aforementioned descriptions concerning conservative variants of the genes and proteins can be applied similarly to variants of any proteins such as L-amino acid biosynthesis system enzymes and genes encoding them.

### <1-3> Methods for increasing activity of protein

Hereinafter, the methods for increasing the activity of a protein will be explained.

The expression "the activity of a protein is increased" means that the activity of the protein is increased as compared with a non-modified strain. Specifically, the expression "the activity of a protein is increased" means that the activity of the protein per cell is increased as compared with that of a non-modified strain. The phrase "non-modified strain" used herein refers to a control strain that has not been modified so that the activity of an objective protein is increased. Examples of the non-modified strain include a wild-type strain and parent strain. Specific examples of the non-modified strain include the respective type strains of the species of bacteria. Specific examples of the non-modified strain also include strains exemplified above in relation to the description of bacteria. That is, in an embodiment, the activity of a protein may be increased as compared with a type strain, namely, the type strain of the species to which the bacterium of the present invention belongs. In another embodiment, the activity of a protein may also be increased as compared with the E. coli K-12 MG1655 strain. In another embodiment, the activity of a protein may also be increased as compared with the P. ananatis AJ13355 strain. In another embodiment, the activity of a protein may also be increased as compared with the P. ananatis NA1 strain. The state that "the activity of a protein is increased" may also be expressed as "the activity of a protein is enhanced". More specifically, the expression "the activity of a protein is increased" may mean that the number of molecules of the protein per cell is increased, and/or the function of each molecule of the protein is increased as compared with those of a non-modified strain. That is, the term "activity" in the expression "the activity of a protein is increased" is not limited to the catalytic activity of the protein and may also mean the transcription amount of a gene (namely, the amount of mRNA) encoding the protein, or the translation amount of the gene (namely, the amount of the protein). The phrase "the number of molecules of a protein per cell" may mean an average value of the number of molecules of the protein per cell. Furthermore, the state that "the activity of a protein is increased" includes not only a state that the activity of an objective protein is increased in a strain inherently having the activity of the objective protein, but also a state that the activity of an objective protein is imparted to a strain not inherently having the activity of the objective protein. Furthermore, so long as the activity of the protein is eventually increased, the activity of an objective protein inherently contained in a host may be attenuated and/or eliminated, and then an appropriate type of the objective protein may be imparted to the host.

The degree of the increase in the activity of a protein is not particularly limited, so long as the activity of the protein is increased as compared with a non-modified strain. The activity of the protein may be increased to, for example, 1.5 times or more, 2 times or more, or 3 times or more of that of a non-modified strain. Furthermore, when the non-modified strain does not have the activity of the objective protein, it is sufficient that the protein is produced as a result of introduction of the gene encoding the protein, and for example, the protein may be produced to such an extent that the activity thereof can be measured.

The modification for increasing the activity of a protein can be attained by, for example, increasing the expression of a gene encoding the protein. The expression "the expression of a gene is increased" means that the expression of the gene is increased as compared with a non-modified strain such as a wild-type strain and parent strain. Specifically, the expression "the expression of a gene is increased" means that the expression amount of the gene per cell is increased as compared with that of a non-modified strain. The phrase "the expression amount of a gene per cell" may mean an average value of the expression amount of the gene per cell. More specifically, the expression "the expression of a gene is increased" may mean that the transcription amount of the gene (namely, the amount of mRNA) is increased, and/or the translation amount of the gene (namely, the amount of the protein expressed from the gene) is increased. The state that "the expression of a gene is increased" may also be referred to as "the expression of a gene is enhanced". The expression of a gene may be increased to, for example, 1.5 times or more, 2 times or more, or 3 times or more of that of a non-modified strain. Furthermore, the state that "the expression of a gene is increased" includes not only a state that the expression amount of an objective gene is increased in a strain that inherently expresses the objective gene, but also a state that the gene is introduced into a strain that does not inherently express the objective gene and expressed therein. That is, the phrase "the expression of a gene is increased" may also mean, for example, that an objective gene is introduced into a strain that does not possess the gene and is expressed therein.

The expression of a gene can be increased by, for example, increasing the copy number of the gene.

The copy number of a gene can be increased by introducing the gene into the chromosome of a host. A gene can be introduced into a chromosome by, for example, using homologous recombination (Miller, J.H., Experiments in Molecular Genetics, 1972, Cold Spring Harbor Laboratory). Examples of the gene transfer method utilizing homologous recombination include, for example, a method of using a linear DNA such as Red-driven integration (Datsenko, K.A., and Wanner, B.L., Proc. Natl. Acad. Sci. USA, 97:6640-6645 (2000)), a method of using a plasmid containing a temperature sensitive replication origin, a method of using a plasmid capable of conjugative transfer, a method of using a suicide vector not having a replication origin that functions in a host, and a transduction method using a phage. Only one copy, or two or more copies of a gene may be introduced. For example, by performing homologous recombination using a nucleotide sequence which is present in multiple copies on a chromosome as a target, multiple copies of a gene can be introduced into the chromosome. Examples of such a nucleotide sequence which is present in multiple copies on a chromosome include repetitive DNAs, and inverted repeats located at both ends of a transposon. Alternatively, homologous recombination may be performed using an appropriate nucleotide sequence on a chromosome such as a gene unnecessary for production of an objective substance as a target. Furthermore, a gene can also be randomly introduced into a chromosome using a transposon or Mini-Mu (Japanese Patent Laid-open (Kokai) No. 2-109985, U.S. Patent No. 5,882,888, EP805867B1). Such methods for modifying a chromosome using homologous recombination can be used for any modification on a chromosome, such as a modification of an expression control sequence, as well as for introduction of an objective gene.

Introduction of an objective gene into a chromosome can be confirmed by Southern hybridization using a probe having a sequence complementary to the whole gene or a part thereof, PCR using primers prepared on the basis of the sequence of the gene, or the like.

Furthermore, the copy number of a gene can also be increased by introducing a vector containing the gene into a host. For example, the copy number of an objective gene can be increased by ligating a DNA fragment containing the objective gene with a vector that functions in a host to construct an expression vector of the gene and transforming the host with the expression vector. The DNA fragment containing the objective gene can be obtained by, for example, PCR using the genomic DNA of a microorganism having the objective gene as the template. As the vector, a vector autonomously replicable in the cell of the host can be used. The vector is preferably a multi-copy vector. Furthermore, the vector preferably has a marker such as an antibiotic resistance gene for selection of transformant. Furthermore, the vector may have a promoter and/or terminator for expressing the introduced gene. The vector may be, for example, a vector derived from a bacterial plasmid, a vector derived from a yeast plasmid, a vector derived from a bacteriophage, cosmid, phagemid, or the like. Specific examples of vector autonomously replicable in Enterobacteriaceae bacteria such as Escherichia coli include, for example, pUC19, pUC18, pHSG299, pHSG399, pHSG398, pBR322, pSTV29 (all of these are available from Takara Bio), pACYC184, pMW219 (NIPPON GENE), pTrc99A (Pharmacia), pPROK series vectors (Clontech), pKK233-2 (Clontech), pET series vectors (Novagen), pQE series vectors (QIAGEN), pCold TF DNA (Takara Bio), pACYC series vectors, and the broad host spectrum vector RSF1010.

When a gene is introduced, it is sufficient that the gene is expressibly harbored by a host. Specifically, it is sufficient that the gene is harbored by a host so that it is expressed under control by a promoter that functions in the host. The promoter is not particularly limited so long as it functions in the host. The phrase "promoter that functions in a host" refers to a promoter that shows a promoter activity in the host. The promoter may be a promoter derived from the host, or a heterogenous promoter. The promoter may be the native promoter of the gene to be introduced, or a promoter of another gene. As the promoter, for example, such a stronger promoter as mentioned later may also be used.

A terminator for termination of gene transcription may be located downstream of the gene. The terminator is not particularly limited so long as it functions in the host. The terminator may be a terminator derived from the host, or a heterogenous terminator. The terminator may be the native terminator of the gene to be introduced, or a terminator of another gene. Specific examples of the terminator include, for example, T7 terminator, T4 terminator, fd phage terminator, tet terminator, and trpA terminator.

Vectors, promoters, and terminators available in various microorganisms are disclosed in detail in "Fundamental Microbiology Vol. 8, Genetic Engineering, KYORITSU SHUPPAN CO., LTD, 1987", and those can be used.

Furthermore, when two or more of genes are introduced, it is sufficient that the genes each are expressibly harbored by the host. For example, all the genes may be carried by a single expression vector or a chromosome. Furthermore, the genes may be separately carried by two or more expression vectors, or separately carried by a single or two or more expression vectors and a chromosome. An operon constituted by two or more genes may also be introduced. The case of "introducing two or more genes" include, for example, cases of introducing respective genes encoding two or more kinds of proteins (such as enzymes), introducing respective genes encoding two or more subunits constituting a single protein complex (such as enzyme complex), and a combination of the foregoing cases.

The gene to be introduced is not particularly limited so long as it encodes a protein that functions in the host. The gene to be introduced may be a gene derived from the host or may be a heterogenous gene. The gene to be introduced can be obtained by, for example, PCR using primers designed on the basis of the nucleotide sequence of the gene in combination with the genomic DNA of an organism having the gene, a plasmid carrying the gene, or the like as a template. The gene to be introduced may also be totally synthesized, for example, on the basis of the nucleotide sequence of the gene (Gene, 60(1), 115-127 (1987)). The obtained gene can be used as it is, or after being modified as required. That is, a gene can be modified to obtain a variant thereof. A gene can be modified by a known technique. For example, an objective mutation can be introduced into an objective site of DNA by the site-specific mutation method. That is, the coding region of a gene can be modified by the site-specific mutation method so that a specific site of the encoded protein include substitution, deletion, insertion, and/or addition of amino acid residues. Examples of the site-specific mutation method include the method utilizing PCR (Higuchi, R., 61, in PCR Technology, Erlich, H.A. Eds., Stockton Press (1989); Carter, P., Meth. in Enzymol., 154, 382 (1987)), and the method utilizing phage (Kramer, W. and Frits, H.J., Meth. in Enzymol., 154, 350 (1987); Kunkel, T.A. et al., Meth. in Enzymol., 154, 367 (1987)). Alternatively, a variant of a gene may be totally synthesized.

Incidentally, when a protein functions as a complex consisting of a plurality of subunits, a part or all of the plurality of subunits may be modified, so long as the activity of the protein is eventually increased. That is, for example, when the activity of a protein is increased by increasing the expression of a gene, the expression of a part or all of the plurality of genes that encode the subunits may be enhanced. It is usually preferable to enhance the expression of all of the plurality of genes encoding the subunits. Furthermore, the subunits constituting the complex may be derived from a single kind of organism or two or more kinds of organisms, so long as the complex has a function of the objective protein. That is, for example, genes of the same organism encoding a plurality of subunits may be introduced into a host, or genes of different organisms encoding a plurality of subunits may be introduced into a host.

Furthermore, the expression of a gene can be increased by improving the transcription efficiency of the gene. In addition, the expression of a gene can also be increased by improving the translation efficiency of the gene. The transcription efficiency of the gene and the translation efficiency of the gene can be improved by, for example, modifying an expression control sequence of the gene. The phrase "expression control sequence" collectively refers to sites that affect the expression of a gene. Examples of the expression control sequence include, for example, promoter, Shine-Dalgarno (SD) sequence (also referred to as ribosome binding site (RBS)), and spacer region between RBS and the start codon. Expression control sequences can be identified using a promoter search vector or gene analysis software such as GENETYX. These expression control sequences can be modified by, for example, a method of using a temperature sensitive vector, or the Red driven integration method (WO2005/010175).

The transcription efficiency of a gene can be improved by, for example, replacing the promoter of the gene on a chromosome with a stronger promoter. The phrase "stronger promoter" refers to a promoter providing an improved transcription of a gene compared with an inherently existing wild-type promoter of the gene. Examples of stronger promoters include, for example, the known high expression promoters such as T7 promoter, trp promoter, lac promoter, thr promoter, tac promoter, trc promoter, tet promoter, araBAD promoter, rpoH promoter, msrA promoter, Pm1 promoter (derived from the genus Bifidobacterium), PR promoter, and PL promoter. Furthermore, as the stronger promoter, a highly-active type of an existing promoter may also be obtained using various reporter genes. For example, by making the -35 and -10 regions in a promoter region closer to the consensus sequence, the activity of the promoter can be enhanced (WO00/18935). Examples of highly active-type promoter include various tac-like promoters (Katashkina JI et al., Russian Federation Patent Application No. 2006134574) and pnlp8 promoter (WO2010/027045). Methods for evaluating the strength of promoters and examples of strong promoters are described in the paper of Goldstein et al. (Prokaryotic Promoters in Biotechnology, Biotechnol. Annu. Rev., 1, 105-128 (1995)), and so forth.

The translation efficiency of a gene can be improved by, for example, replacing the Shine-Dalgarno (SD) sequence (also referred to as ribosome binding site (RBS)) for the gene on a chromosome with a stronger SD sequence. The "stronger SD sequence" means a SD sequence that provides an improved translation of mRNA compared with the inherently existing wild-type SD sequence of the gene. Examples of stronger SD sequences include, for example, RBS of the gene 10 derived from phage T7 (Olins P.O. et al, Gene, 1988, 73, 227-235). Furthermore, it is known that substitution, insertion, or deletion of several nucleotides in a spacer region between RBS and the start codon, especially in a sequence immediately upstream of the start codon (5'-UTR), significantly affects the stability and translation efficiency of mRNA, and hence, the translation efficiency of a gene can also be improved by modifying them.

The translation efficiency of a gene can also be improved by, for example, modifying codons. For example, the translation efficiency of the gene can be improved by replacing a rare codon present in the gene with a synonymous codon more frequently used. That is, the gene to be introduced may be modified, for example, to contain optimal codons according to the frequencies of codons observed in a host to be used. Codons can be replaced by, for example, the site-specific mutation method. Alternatively, a gene fragment in which objective codons are replaced may be totally synthesized. Frequencies of codons in various organisms are disclosed in the "Codon Usage Database" (http://www.kazusa.or.jp/codon; Nakamura, Y. et al, Nucl. Acids Res., 28, 292 (2000)).

Furthermore, the expression of a gene can also be increased by amplifying a regulator that increases the expression of the gene, or deleting or attenuating a regulator that reduces the expression of the gene.

Such methods for increasing the gene expression as mentioned above may be used independently or in any appropriate combination.

Furthermore, the modification that increases the activity of a protein can also be attained by, for example, enhancing the specific activity of the protein. Enhancement of the specific activity also includes desensitization to feedback inhibition. A protein having an enhanced specific activity can be obtained by, for example, searching various organisms. Furthermore, a highly-active type of an existing protein may also be obtained by introducing a mutation into the existing protein. The mutation to be introduced may be, for example, substitution, deletion, insertion, and/or addition of one or several amino acid residues at one or several position of the protein. The mutation can be introduced by, for example, such a site-specific mutation method as mentioned above. The mutation may also be introduced by, for example, a mutagenesis treatment. Examples of the mutagenesis treatment include irradiation of X-ray, irradiation of ultraviolet, and a treatment with a mutation agent such as N-methyl-N'-nitro-N-nitrosoguanidine (MNNG), ethyl methanesulfonate (EMS), and methyl methanesulfonate (MMS). Furthermore, a random mutation may be induced by directly treating DNA in vitro with hydroxylamine. Enhancement of the specific activity may be independently used or may be used in any appropriate combination with such methods for enhancing gene expression as mentioned above.

The method for the transformation is not particularly limited, and conventionally known methods can be used. There can be used, for example, a method of treating recipient cells with calcium chloride to increase the permeability thereof for DNA, which has been reported for the Escherichia coli K-12 strain (Mandel, M. and Higa, A., J. Mol. Biol., 1970, 53, 159-162), and a method of preparing competent cells from cells which are in the growth phase, followed by transformation with DNA, which has been reported for Bacillus subtilis (Duncan, C.H., Wilson, G.A. and Young, F.E., Gene, 1977, 1:153-167). Alternatively, there can also be used a method of making DNA-recipient cells into protoplasts or spheroplasts, which can easily take up recombinant DNA, followed by introducing a recombinant DNA into the DNA-recipient cells, which is known to be applicable to Bacillus subtilis, actinomycetes, and yeasts (Chang, S. and Choen, S.N., 1979, Mol. Gen. Genet., 168:111-115; Bibb, M.J., Ward, J.M. and Hopwood, O.A., 1978, Nature, 274:398-400; Hinnen, A., Hicks, J.B. and Fink, G.R., 1978, Proc. Natl. Acad. Sci. USA, 75:1929-1933). Furthermore, the electric pulse method reported for coryneform bacteria (Japanese Patent Laid-open (Kokai) No. 2-207791) can also be used.

An increase in the activity of a protein can be confirmed by measuring the activity of the protein.

An increase in the activity of a protein can also be confirmed by confirming an increase in the expression of a gene encoding the protein. An increase in the expression of a gene can be confirmed by confirming an increase in the transcription amount of the gene, or by confirming an increase in the amount of a protein expressed from the gene.

An increase of the transcription amount of a gene can be confirmed by comparing the amount of mRNA transcribed from the gene with that of a non-modified strain such as a wild-type strain or parent strain. Examples of the method for evaluating the amount of mRNA include Northern hybridization, RT-PCR, microarray, RNA-seq, and so forth (Sambrook, J., et al., Molecular Cloning A Laboratory Manual/Third Edition, Cold Spring Harbor Laboratory Press, Cold Spring Harbor (USA), 2001). The amount of mRNA (such as the number of molecules of the mRNA per cell) may be increased to, for example, 1.5 times or more, 2 times or more, or 3 times or more of that of a non-modified strain.

An increase in the amount of a protein can be confirmed by Western blotting using antibodies (Sambrook, J., et al., Molecular Cloning A Laboratory Manual/Third Edition, Cold Spring Harbor Laboratory Press, Cold Spring Harbor (USA), 2001). The amount of the protein (such as the number of molecules of the protein per cell) may be increased to, for example, 1.5 times or more, 2 times or more, or 3 times or more of that of a non-modified strain.

The aforementioned methods for increasing the activity of a protein can be used for enhancement of the activities of any proteins and enhancement of the expression of any genes.

### <1-4> Method for reducing activity of protein

Hereinafter, the methods for reducing the activity of a protein will be explained.

The expression "the activity of a protein is reduced" means that the activity of the protein is reduced as compared with a non-modified strain. Specifically, the expression "the activity of a protein is reduced" means that the activity of the protein per cell is reduced as compared with that of a non-modified strain. The phrase "non-modified strain" used herein refers to a control strain that has not been modified so that the activity of an objective protein is reduced. Examples of the non-modified strain include a wild-type strain and parent strain. Specific examples of the non-modified strain include the respective type strains of the species of bacteria. Specific examples of the non-modified strain also include strains exemplified above in relation to the description of bacteria. That is, in an embodiment, the activity of a protein may be reduced as compared with a type strain, namely, the type strain of the species to which the bacterium of the present invention belongs. In another embodiment, the activity of a protein may also be reduced as compared with the E. coli K-12 MG1655 strain. In another embodiment, the activity of a protein may also be reduced as compared with the P. ananatis AJ13355 strain. In another embodiment, the activity of a protein may also be reduced as compared with the P. ananatis NA1 strain. The state that "the activity of a protein is reduced" also includes a state that the activity of the protein has completely disappeared. More specifically, the expression "the activity of a protein is reduced" may mean that the number of molecules of the protein per cell is reduced, and/or the function of each molecule of the protein is reduced as compared with those of a non-modified strain. That is, the term "activity" in the expression "the activity of a protein is reduced" is not limited to the catalytic activity of the protein and may also mean the transcription amount of a gene (namely, the amount of mRNA) encoding the protein or the translation amount of the gene (namely, the amount of the protein). The phrase "the number of molecules of a protein per cell" may mean an average value of the number of molecules of the protein per cell. The state that "the number of molecules of the protein per cell is reduced" also includes a state that the protein does not exist at all. The state that "the function of each molecule of the protein is reduced" also includes a state that the function of each protein molecule has completely disappeared. The degree of the reduction in the activity of a protein is not particularly limited, so long as the activity is reduced as compared with that of a non-modified strain. The activity of a protein may be reduced to, for example, 50% or less, 20% or less, 10% or less, 5% or less, or 0% of that of a non-modified strain.

The modification for reducing the activity of a protein can be attained by, for example, reducing the expression of a gene encoding the protein. The expression "the expression of a gene is reduced" means that the expression of the gene is reduced as compared with a non-modified strain such as a wild-type strain and parent strain. Specifically, the expression "the expression of a gene is reduced" means that the expression of the gene per cell is reduced as compared with that of a non-modified strain. The phrase "the expression amount of a gene per cell" may mean an average value of the expression amount of the gene per cell. More specifically, the expression "the expression of a gene is reduced" may mean that the transcription amount of the gene (namely, the amount of mRNA) is reduced, and/or the translation amount of the gene (namely, the amount of the protein expressed from the gene) is reduced. The state that "the expression of a gene is reduced" also includes a state that the gene is not expressed at all. The state that "the expression of a gene is reduced" is also referred to as "the expression of a gene is attenuated". The expression of a gene may be reduced to, for example, 50% or less, 20% or less, 10% or less, 5% or less, or 0% of that of a non-modified strain.

The reduction in gene expression may be due to, for example, a reduction in the transcription efficiency, a reduction in the translation efficiency, or a combination of them. The expression of a gene can be reduced by modifying an expression control sequence of the gene such as a promoter, Shine-Dalgarno (SD) sequence (also referred to as ribosome-binding site (RBS)), and spacer region between RBS and the start codon of the gene. When an expression control sequence is modified, preferably one or more nucleotides, more preferably two or more nucleotides, particularly preferably three or more nucleotides, of the expression control sequence are modified. The transcription efficiency of a gene can be reduced by, for example, replacing the promoter of the gene on a chromosome with a weaker promoter. The phrase "weaker promoter" means a promoter providing an attenuated transcription of a gene compared with an inherently existing wild-type promoter of the gene. Examples of weaker promoters include, for example, inducible promoters. That is, an inducible promoter may function as a weaker promoter under a non-induced condition, such as in the absence of the corresponding inducer. Furthermore, a partial or entire region of an expression control sequence may be deleted. The expression of a gene can also be reduced by, for example, manipulating a factor responsible for expression control. Examples of the factor responsible for expression control include low molecules responsible for transcription or translation control (inducers, inhibitors, etc.), proteins responsible for transcription or translation control (transcription factors etc.), nucleic acids responsible for transcription or translation control (siRNA etc.), and so forth. Furthermore, the expression of a gene can also be reduced by, for example, introducing a mutation that reduces the expression of the gene into the coding region of the gene. For example, the expression of a gene can be reduced by replacing a codon in the coding region of the gene with a synonymous codon used less frequently in a host. Furthermore, for example, the gene expression may be reduced due to disruption of a gene as described below.

The modification for reducing the activity of a protein can also be attained by, for example, disrupting a gene encoding the protein. The expression "a gene is disrupted" means that a gene is modified so that a protein that can normally function is not produced. The state that "a protein that normally functions is not produced" includes a state that the protein is not produced at all from the gene, and a state that the protein of which the function (such as activity or property) per molecule is reduced or eliminated is produced from the gene.

Disruption of a gene can be attained by, for example, deleting the gene on a chromosome. The phrase "deletion of a gene" refers to deletion of a partial or entire region of the coding region of the gene. Furthermore, the whole of a gene including sequences upstream and downstream from the coding region of the gene on a chromosome may be deleted. Sequences upstream and downstream from the coding region of a gene may contain, for example, an expression control sequence of the gene. The region to be deleted may be any region such as an N-terminal region (region encoding an N-terminal region of a protein), an internal region, or a C-terminal region (region encoding a C-terminal region of a protein), so long as the activity of the protein can be reduced. Deletion of a longer region can usually more surely inactivate the gene. The region to be deleted may be, for example, a region having a length of 10% or more, 20% or more, 30% or more, 40% or more, 50% or more, 60% or more, 70% or more, 80% or more, 90% or more, or 95% or more of the total length of the coding region of the gene. Furthermore, it is preferred that reading frames of the sequences upstream and downstream from the region to be deleted are not the same. Inconsistency of reading frames may cause a frameshift downstream of the region to be deleted.

Disruption of a gene can also be attained by, for example, introducing an amino acid substitution (missense mutation), a stop codon (nonsense mutation), addition or deletion of one or two nucleotide residues (frame shift mutation), or the like into the coding region of the gene on a chromosome (Journal of Biological Chemistry, 272:8611-8617 (1997); Proceedings of the National Academy of Sciences, USA, 95 5511-5515 (1998); Journal of Biological Chemistry, 26 116, 20833-20839 (1991)).

Disruption of a gene can also be attained by, for example, inserting another nucleotide sequence into a coding region of the gene on a chromosome. Site of the insertion may be in any region of the gene, and insertion of a longer nucleotide sequence can usually more surely inactivate the gene. It is preferred that reading frames of the sequences upstream and downstream from the insertion site are not the same. Inconsistency of reading frames may cause a frameshift downstream of the insertion site. The other nucleotide sequence is not particularly limited so long as a sequence that reduces or eliminates the activity of the encoded protein is chosen, and examples thereof include, for example, a marker gene such as antibiotic resistance genes, and a gene useful for production of an objective substance.

Particularly, disruption of a gene may be carried out so that the amino acid sequence of the encoded protein is deleted. In other words, the modification for reducing the activity of a protein can be attained by, for example, deleting the amino acid sequence of the protein, specifically, modifying a gene to encode a protein of which the amino acid sequence is deleted. The term "deletion of the amino acid sequence of a protein" refers to deletion of a partial or entire region of the amino acid sequence of the protein. In addition, the term "deletion of the amino acid sequence of a protein" means that the original amino acid sequence disappears in the protein, and includes when the original amino acid sequence is changed to another amino acid sequence. That is, for example, a region that was changed to another amino acid sequence by frameshift may be regarded as a deleted region. When the amino acid sequence of a protein is deleted, the total length of the protein is typically shortened, but there can also be cases where the total length of the protein is not changed or is extended. For example, by deletion of a partial or entire region of the coding region of a gene, a region encoded by the deleted region can be deleted in the encoded protein. In addition, for example, by introduction of a stop codon into the coding region of a gene, a region encoded by the downstream region of the introduction site can be deleted in the encoded protein. In addition, for example, by frameshift in the coding region of a gene, a region encoded by the frameshift region can be deleted in the encoded protein. The aforementioned descriptions concerning the position and length of the region to be deleted in deletion of a gene can be applied similarly to the position and length of the region to be deleted in deletion of the amino acid sequence of a protein.

Such modification of a gene on a chromosome as described above can be attained by, for example, preparing a disruption-type gene modified so that it is unable to produce a protein that normally functions, and transforming a host with a recombinant DNA containing the disruption-type gene to cause homologous recombination between the disruption-type gene and the wild-type gene on a chromosome and thereby substitute the disruption-type gene for the wild-type gene on the chromosome. In this procedure, if a marker gene selected according to the characteristics of the host such as auxotrophy is included in the recombinant DNA, the operation becomes easier. Examples of the disruption-type gene include a gene of which a partial or entire region of the coding region is deleted, gene including a missense mutation, gene including a nonsense mutation, gene including a frame shift mutation, and gene introduced with an insertion sequence such as a transposon or marker gene. The protein encoded by the disruption-type gene has a conformation different from that of the wild-type protein, even if it is produced, and thus the function thereof is reduced or eliminated. The structure of the recombinant DNA to be used for homologous recombination is not particularly limited, so long as homologous recombination occurs in a desired manner. For example, a host can be transformed with a linear DNA containing a disruption-type gene and having nucleotide sequences upstream and downstream of a wild-type gene on the chromosome at the respective ends, to thereby cause homologous recombination at both upstream and downstream of the wild-type gene, thereby resulting in replacement of the wild-type gene with the disruption-type gene. Such gene disruption based on gene substitution utilizing homologous recombination has already been established, and there are methods of using a linear DNA such as a method called "Red driven integration" (Datsenko, K.A, and Wanner, B.L., Proc. Natl. Acad. Sci. USA, 97:6640-6645 (2000)), and a method utilizing the Red driven integration in combination with an excision system derived from λ phage (Cho, E.H., Gumport, R.I., Gardner, J.F., J. Bacteriol., 184:5200-5203 (2002)) (refer to WO2005/010175), a method of using a plasmid having a temperature sensitive replication origin, a method of using a plasmid capable of conjugative transfer, a method of utilizing a suicide vector not having a replication origin that functions in a host (U.S. Patent No. 6,303,383, Japanese Patent Laid-open (Kokai) No. 05-007491), and so forth. Such methods for modifying a chromosome using homologous recombination can be used for any modification on a chromosome, such as a modification of an expression control sequence, as well as for disruption of an objective gene.

Modification for reducing activity of a protein can also be attained by, for example, a mutagenesis treatment. Examples of the mutagenesis treatment include irradiation of X-ray or ultraviolet and treatment with a mutation agent such as N-methyl-N'-nitro-N-nitrosoguanidine (MNNG), ethyl methanesulfonate (EMS), and methyl methanesulfonate (MMS).

Such methods for reducing the activity of a protein as mentioned above may be used independently or in any appropriate combination.

When a protein functions as a complex consisting of a plurality of subunits, a part or all of the plurality of subunits may be modified, so long as the activity of the protein is eventually reduced. That is, for example, a part or all of a plurality of genes that encode the respective subunits may be disrupted or the like. Furthermore, when there is a plurality of isozymes of a protein, a part or all of the activities of the plurality of isozymes may be reduced, so long as the activity of the protein is eventually reduced. That is, for example, a part or all of a plurality of genes that encode the respective isozymes may be disrupted or the like.

A reduction in the activity of a protein can be confirmed by measuring the activity of the protein.

A reduction in the activity of a protein can also be confirmed by confirming a reduction in the expression of a gene encoding the protein. A reduction in the expression of a gene can be confirmed by confirming a reduction in the transcription amount of the gene or a reduction in the amount of the protein expressed from the gene.

A reduction in the transcription amount of a gene can be confirmed by comparing the amount of mRNA transcribed from the gene with that of a non-modified strain. Examples of the method for evaluating the amount of mRNA include Northern hybridization, RT-PCR, microarray, RNA-seq, and so forth (Sambrook, J., et al., Molecular Cloning A Laboratory Manual/Third Edition, Cold Spring Harbor Laboratory Press, Cold Spring Harbor (USA), 2001). The amount of mRNA (such as the number of molecules of the mRNA per cell) may be reduced to, for example, 50% or less, 20% or less, 10% or less, 5% or less, or 0% of that of a non-modified strain.

A reduction in the amount of a protein can be confirmed by Western blotting using antibodies (Sambrook, J., et al., Molecular Cloning A Laboratory Manual/Third Edition, Cold Spring Harbor Laboratory Press, Cold Spring Harbor (USA), 2001). The amount of the protein (such as the number of molecules of the protein per cell) may be reduced to, for example, 50% or less, 20% or less, 10% or less, 5% or less, or 0% of that of a non-modified strain.

Disruption of a gene can be confirmed by determining nucleotide sequence of a part or the whole of the gene, restriction enzyme map, full length, or the like of the gene depending on the means used for the disruption.

The aforementioned methods for reducing the activity of a protein can be applied to reduction in the activities of any proteins and reduction in the expression and disruption of any genes. The aforementioned methods for reducing the activity of a protein can be applied even when the gene whose expression is to be reduced or which is to be disrupted is present on a structure outside the chromosome, such as a plasmid. In such cases, the term "chromosome" in the aforementioned methods for reducing the activity of a protein may be read as "structure outside a chromosome", such as a plasmid.

### <2> Method for producing L-amino acid of the present invention

The method of the present invention is a method for producing an L-amino acid comprising culturing the bacterium of the present invention in a culture medium to accumulate the L-amino acid in the culture medium and/or cells of the bacterium, and collecting the L-amino acid from the culture medium and/or cells of the bacterium. The L-amino acid is as described above. In the present invention, one kind of L-amino acid may be produced, or two or more kinds of L-amino acids may be produced.

The culture medium to be used is not particularly limited, so long as the bacterium of the present invention can proliferate in it, and an objective L-amino acid can be produced. As the culture medium, for example, a usual culture medium used for culture of bacteria such as Enterobacteriaceae bacteria can be used. As the culture medium, for example, a culture medium containing carbon source, nitrogen source, phosphorus source, and sulfur source, as well as components selected from other various organic components and inorganic components as required can be used. Types and concentrations of the culture medium components can be appropriately determined according to various conditions such as the type of bacterium to be used.

Specific examples of the carbon source include, for example, saccharides such as glucose, fructose, sucrose, lactose, galactose, xylose, arabinose, blackstrap molasses, hydrolysates of starches, and hydrolysates of biomass, organic acids such as acetic acid, fumaric acid, citric acid, and succinic acid, alcohols such as glycerol, crude glycerol, and ethanol, and aliphatic acids. Particular examples of the carbon source include saccharides. As the carbon source, plant-derived materials can be preferably used. Examples of the plant include, for example, corn, rice, wheat, soybean, sugarcane, beet, and cotton. Examples of the plant-derived materials include, for example, organs such as root, stem, trunk, branch, leaf, flower, and seed, plant bodies including them, and decomposition products of these plant organs. The forms of the plant-derived materials at the time of use thereof are not particularly limited, and they can be used in any form such as unprocessed product, juice, ground product, and purified product. Pentoses such as xylose, hexoses such as glucose, or mixtures of them can be obtained from, for example, plant biomass, and used. Specifically, these saccharides can be obtained by subjecting a plant biomass to such a treatment as steam treatment, hydrolysis with concentrated acid, hydrolysis with diluted acid, hydrolysis with an enzyme such as cellulase, and alkaline treatment. Since hemicellulose is generally more easily hydrolyzed compared with cellulose, hemicellulose in a plant biomass may be hydrolyzed beforehand to liberate pentoses, and then cellulose may be hydrolyzed to generate hexoses. Furthermore, xylose may be supplied by conversion from hexoses by, for example, imparting a pathway for converting hexose such as glucose to xylose to the bacterium of the present invention. As the carbon source, a single kind of carbon source may be used, or two or more kinds of carbon sources may be used in combination.

Specific examples of the nitrogen source include, for example, ammonium salts such as ammonium sulfate, ammonium chloride, and ammonium phosphate, organic nitrogen sources such as peptone, yeast extract, meat extract, and soybean protein decomposition products, ammonia, and urea. Ammonia gas or aqueous ammonia used for adjusting pH may also be used as the nitrogen source. As the nitrogen source, a single kind of nitrogen source may be used, or two or more kinds of nitrogen sources may be used in combination.

Specific examples of the phosphate source include, for example, phosphoric acid salts such as potassium dihydrogenphosphate and dipotassium hydrogenphosphate, and phosphoric acid polymers such as pyrophosphoric acid. As the phosphate source, a single kind of phosphate source may be used, or two or more kinds of phosphate sources may be used in combination.

Specific examples of the sulfur source include, for example, inorganic sulfur compounds such as sulfates, thiosulfates, and sulfites, and sulfur-containing amino acids such as cysteine, cystine, and glutathione. As the sulfur source, a single kind of sulfur source may be used, or two or more kinds of sulfur sources may be used in combination.

Specific examples of other various organic components and inorganic components include, for example, inorganic salts such as sodium chloride and potassium chloride; trace metals such as iron, manganese, magnesium, calcium, zinc, copper, cobalt; vitamins such as vitamin B 1, vitamin B2, vitamin B6, nicotinic acid, nicotinamide, and vitamin B 12; amino acids; nucleic acids; and organic components containing those such as peptone, casamino acid, yeast extract, and soybean protein decomposition product. As other various organic components and inorganic components, a single kind of component may be used, or two or more kinds of components may be used in combination.

Furthermore, when an auxotrophic mutant that requires an amino acid or the like for growth thereof is used, it is preferable to supply a required nutrient to the culture medium.

Furthermore, it is also preferable to, for example, restrict the amount of biotin in the culture medium, or add a surfactant or penicillin to the culture medium.

The culture conditions are not particularly limited so long as the bacterium of the present invention can proliferate, and the objective L-amino acid can be produced. The culture can be performed, for example, under usual conditions used for culturing bacteria such as Enterobacteriaceae bacteria. The culture conditions can be appropriately set according to various conditions such as the type of bacterium to be used.

The culture can be performed using a liquid culture medium. At the time of the culture, the bacterium of the present invention cultured on a solid culture medium such as agar medium may be directly inoculated into a liquid culture medium, or the bacterium of the present invention cultured in a liquid culture medium as seed culture may be inoculated into a liquid culture medium for main culture. That is, the culture may be performed separately as seed culture and main culture. In such a case, the culture conditions of the seed culture and the main culture may be or may not be the same. Amount of the bacterium of the present invention contained in the culture medium at the time of the start of the culture is not particularly limited. The main culture may be performed by, for example, inoculating a seed culture broth to a culture medium for main culture at an amount of 1 to 50%(v/v).

The culture can be performed as batch culture, fed-batch culture, continuous culture, or a combination of these. The culture medium used at the time of the start of the culture is also referred to as "starting medium". The culture medium supplied to a culture system (fermentation tank) in fed-batch culture or continuous culture is also referred to as "feed medium". Furthermore, to supply a feed medium to a culture system in fed-batch culture or continuous culture is also referred to as to "feed". Furthermore, when the culture is performed separately as seed culture and main culture, for example, both the seed culture and the main culture may be performed as batch culture. Alternatively, for example, the seed culture may be performed as batch culture, and the main culture may be performed as fed-batch culture or continuous culture.

In the present invention, the culture medium components each may be contained in the starting medium, feed medium, or both. The types of the components contained in the starting medium may be or may not be the same as the types of the components contained in the feed medium. The concentration of each component contained in the starting medium may be or may not be the same as the concentration of the component contained in the feed medium. Furthermore, two or more kinds of feed media containing different types and/or different concentrations of components may be used. For example, when the feed medium is intermittently fed a plurality of times, the types and/or concentrations of components contained in the feed media may be or may not be the same for each feeding.

The concentration of the carbon source in the culture medium is not particularly limited, so long as the bacterium of the present invention can proliferate and produce the L-amino acid. The concentration of the carbon source in the culture medium may be as high as possible within such a range that production of the L-amino acid is not inhibited. The concentration of the carbon source in the culture medium may be, as the initial concentration (the concentration in the starting medium), for example, 1 to 30% (w/v), preferably 3 to 10% (w/v). Furthermore, the carbon source may be additionally supplied to the culture medium as required. For example, the carbon source may be additionally supplied to the culture medium in proportion to consumption of the carbon source accompanying progress of the fermentation.

The culture can be performed, for example, under an aerobic condition. The phrase "aerobic condition" refers to a condition where the dissolved oxygen concentration in the liquid culture medium is not lower than 0.33 ppm, which is the detection limit for the detection with an oxygen membrane electrode, or may preferably to a condition where the dissolved oxygen concentration in the liquid culture medium is not lower than 1.5 ppm. The oxygen concentration can be controlled to, for example, 5 to 50%, preferably about 10%, of the saturated oxygen concentration. Specifically, the culture under an aerobic condition can be performed by aeration culture, shaking culture, stirring culture, or a combination thereof. The pH of the culture medium may be, for example, 3 to 10, preferably 4.0 to 9.5. During the culture, the pH of the culture medium can be adjusted as required. The pH of the culture medium can be adjusted using various alkaline and acidic substances such as ammonia gas, aqueous ammonia, sodium carbonate, sodium bicarbonate, potassium carbonate, potassium bicarbonate, magnesium carbonate, sodium hydroxide, potassium hydroxide, calcium hydroxide, and magnesium hydroxide. The culture temperature may be, for example, 20 to 40°C, preferably 25 to 37°C. The culture period may be, for example, 10 to 120 hours. The culture may be continued, for example, until the carbon source contained in the culture medium is consumed, or until the bacterium of the present invention loses the activity. By culturing the bacterium of the present invention under such conditions as described above, the L-amino acid is accumulated in the culture medium and/or cells of the bacterium.

Moreover, when L-glutamic acid is produced, the culture can be performed using a liquid culture medium adjusted to satisfy a condition under which L-glutamic acid is precipitated, while precipitating L-glutamic acid in the culture medium. Examples of the condition under which L-glutamic acid is precipitated include, for example, pH 5.0 to 4.0, preferably pH 4.5 to 4.0, more preferably pH 4.3 to 4.0, particularly preferably around pH 4.0 (EP1078989A). When using a liquid culture medium adjusted to satisfy a condition under which L-glutamic acid is precipitated, L-glutamic acid can be more efficiently crystalized by adding pantothenic acid into the culture medium (WO2004/111258). Also, when using a liquid culture medium adjusted to satisfy a condition under which L-glutamic acid is precipitated, L-glutamic acid can be more efficiently crystalized by adding L-glutamic acid crystals as seed crystals into the culture medium (EP1233069A). Also, when using a liquid culture medium adjusted to satisfy a condition under which L-glutamic acid is precipitated, L-glutamic acid can be more efficiently crystalized by adding L-glutamic acid crystals and L-lysine crystals as seed crystals into the culture medium (EP1624069A).

For producing a basic amino acid such as L-lysine, the culture step (fermentation step) may be carried out so that bicarbonate ions and/or carbonate ions serve as counter ions for the basic amino acid. Such a fermentation mode is also referred to as "carbonate fermentation". By the carbonate fermentation, a basic amino acid can be produced by fermentation while reducing the amounts of sulfate ions and/or chloride ions to be used, which have been conventionally used as counter ions for a basic amino acid. The carbonate fermentation can be carried out, for example, as described in US2002-025564A, EP1813677A, and JP2002-65287A.

The fermentation broth can be processed using, for example, a hydrocyclone. As the hydrocyclone, for example, one having an ordinary shape, having a cylindrical part having a diameter of 10 to 110 mm, and made of ceramic, stainless steel, or resin can be used. The feeding amount of the fermentation broth to the hydrocyclone can be set depending on, for example, the cell concentration and the L-amino acid concentration in the fermentation broth. The feeding amount of the fermentation broth to the hydrocyclone may be, for example, 2 to 1200 L/min.

Production of the L-amino acid can be confirmed by known methods used for detection or identification of compounds. Examples of such methods include, for example, HPLC, LC/MS, GC/MS, and NMR. These methods can be independently used or can be used in an appropriate combination.

The L-amino acid can be collected from the fermentation broth by known methods used for separation and purification of compounds. Examples of such methods include, for example, ion-exchange resin method (Nagai, H. et al., Separation Science and Technology, 39(16), 3691-3710), precipitation, membrane separation (Japanese Patent Laid-open (Kokai) No. 9-164323 and Japanese Patent Laid-open (Kokai) No. 9-173792), and crystallization (WO2008/078448 and WO2008/078646). These methods can be independently used or can be used in an appropriate combination. When the L-amino acid is accumulated in cells of the bacterium, for example, the cells can be disrupted with ultrasonic waves or the like, a supernatant can be obtained by removing the cells from the cell-disrupted suspension by centrifugation, and the L-amino acid can be collected from the supernatant by the ion exchange resin method or the like. The L-amino acid to be collected may be a free compound, a salt thereof, or a mixture thereof. Examples of the salt include, for example, sulfate, hydrochloride, carbonate, ammonium salt, sodium salt, and potassium salt. When L-glutamic acid is produced, L-glutamic acid to be collected may specifically be, for example, free L-glutamic acid, sodium L-glutamate (such as monosodium L-glutamate, MSG), ammonium L-glutamate (such as monoammonium L-glutamate), or a mixture of these. For example, monosodium L-glutamate (MSG) can be obtained by adding an acid to the fermentation broth to crystallize ammonium L-glutamate contained therein, and then by adding an equimolar of sodium hydroxide to the crystals. In addition, decolorization can be performed using activated carbon before and/or after the crystallization (see, Tetsuya KAWAKITA, "Industrial Crystallization for Monosodium L-Glutamate. ", Bulletin of the Society of Sea Water Science, Japan, Vol.56:5). The monosodium L-glutamate crystal can be used as, for example, an umami seasoning. The monosodium L-glutamate crystal may also be used as a seasoning in combination with a nucleic acid such as sodium guanylate and sodium inosinate, which also have umami taste.

When the L-amino acid is precipitated in the culture medium, it can be collected by centrifugation, filtration, or the like. The L-amino acid precipitated in the culture medium may also be isolated together with the L-amino acid dissolving in the culture medium, after the L-amino acid dissolving in the culture medium is crystallized.

The collected L-amino acid may contain such components as bacterial cells, culture medium components, moisture, and by-product metabolites of the bacterium in addition to the L-amino acid. The collected L-amino acid may also be purified at a desired extent. Purity of the collected L-amino acid may be, for example, 50% (w/w) or higher, preferably 85% (w/w) or higher, particularly preferably 95% (w/w) or higher (JP1214636B, USP5,431,933, USP4,956,471, USP4,777,051, USP4,946,654, USP5,840,358, USP6,238,714, and US2005/0025878).

### Examples

Hereinafter, the present invention will be more specifically explained with reference to non-limiting examples.

### Example: L-glutamic acid production using Pantoea ananatis budABC gene-deletion strain and GlcNDH (PAJ_3461, PAJ 3462, and PAJ_3463) gene-deletion strain

### (1) Materials and methods

### (1-1) Strains used

Pantoea ananatis SC17(0)/RSF-Red-TER strain (WO2008/090770)
Pantoea ananatis NA1 strain (WO2008/090770)

### (1-2) Culture media used

LB medium (Table 1)
Glu fermentation medium (Table 2)
SOC medium (Table 3)
LBGM9 medium (Table 4)

**Table 1. LB medium**

| Component | Final concentration |
|---|---|
| Bacto-tryptone | 10 g/l |
| Bacto-yeast extract | 5 g/l |
| NaCl | 10 g/l |

Agar (15 g/L) was added thereto if the medium was used as a plate.

The medium was sterilized by autoclaving at 120°C for 20 minutes.

After the autoclaving, antibiotic(s) was/were added thereto as required.

**Table 2. Glu fermentation medium**

| < Group A > | |
|---|---|
| Component | Final concentration |
| Ammonium sulfate | 20 g/L |
| KH₂PO₄ | 2 g/L |
| Yeast extract | 2 g/L |
| L-Lys | 0.2 g/L |
| L-Met | 0.2 g/L |
| Diaminopimelic acid | 0.2 g/L |
| FeSO₄·7H₂O | 20 mg/L |
| MnSO₄·7H₂O | 20 mg/L |

This group was adjusted to pH7.0 with KOH and sterilized by autoclaving at 115°C for 10 minutes.

### < Group B >

| Component | Final concentration |
|---|---|
| Glucose | 30 g/L |
| MgSO₄·7H₂O | 0.5 g/L |

This group was sterilized by autoclaving at 115°C for 10 minutes.

### < Group C >

| Component | Final concentration |
|---|---|
| Calcium carbonate | 20 g/L |

This group was sterilized by dry heating at 180°C for 3 hours or longer.

After the sterilization, Groups A, B, and C were mutually mixed, and antibiotic(s) was/were added thereto as required.

**Table 3. SOC medium**

| Component | Final concentration | |
|---|---|---|
| Bacto Trypton | 20 | g/L |
| Yeast Extract | 5 | g/L |
| NaCl | 0.5 | g/L |
| 6N NaOH | 0.167 | mL/L |

The medium was sterilized by autoclaving at 120°C for 20 minutes.

After the autoclaving, the following reagents were added thereto.

| Component | Final concentration |
|---|---|
| 1M Glucose | 20 mL/L |
| 1M MgCl₂ | 10 mL/L |
| 1M MgSO₄ | 10 mL/L |

**Table 4. LBGM9 agar medium**

| Component | Compound amount | |
|---|---|---|
| 1.1-fold concentrated LB medium | 270 | mL |
| 50% Glucose | 3 | mL |
| 10-fold concentrated M9 minimal salts | 30 | mL |
| Agar | 4.5 | g |

The medium was sterilized by autoclaving at 120°C for 20 minutes.

After the autoclaving, antibiotic(s) was/were added thereto as required.

### (1-3) Gene deletion by λ-red method

Pantoea ananatis SC17(0)/RSF-Red-TER strain was inoculated into 4 mL of LB medium containing 25 mg/L Cm (chloramphenicol) in a test tube and cultured overnight at 34°C, 120 rpm with shaking, to obtain a pre-culture broth. Next, 0.5 mL of the pre-culture broth was inoculated into 50 mL of LB medium containing 1 mM IPTG and 25 mg/L Cm in a Sakaguchi flask and cultured for 3 hours. Cells were collected from the culture broth obtained with a cooling centrifuge and washed three times with ice-cold 10% glycerol aqueous solution, to prepare competent cells. Then, a PCR fragment for gene deletion was introduced into the competent cells by electroporation method. SOC medium was added, recovery culture was carried out at 34°C for 2 hours, and then the culture broth was applied to LB agar medium containing 40 mg/L Km (kanamycin). A gene-deletion strain was obtained as a Km-resistant strain.

### (1-4) Gene deletion by genome transfer

Genome was extracted from the gene-deletion strain obtained in (1-3) using a genome extraction kit PurElute^{™} Bacterial Genomic Kit (EdgeBio). Next, a strain to which the gene deletion was to be introduced was cultured overnight on agar medium, and cells were scraped off from the grown plate and washed three times with 10% glycerol, to prepare competent cells. Then, the genome of the gene-deletion strain was introduced into the competent cells by electroporation method. SOC medium was added, recovery culture was carried out at 34°C for 2 hours, and then the culture broth was applied to LBGM9 agar medium containing 40 mg/L Km. A gene-deletion strain was obtained as a Km-resistant strain.

### (2) Construction of P. ananatis NA1ΔbudABC::Km strain

PCR was carried out using genome of Pantoea ananatis SC17(0)hisD::(attL_{λ}-Km^{R}-attR_{λ}) (Joanna I Katashkina et al., Use of the lambda Red-recombineering method for genetic engineering of Pantoea ananatis, BMC Mol Biol, doi: 10.1186/1471-2199-10-34.) as a template in combination with primers of SEQ ID NOS: 13 and 14, to obtain an attL-Km-attR fragment for deletion of budABC genes, which fragment had been added with an upstream sequence of budA ORF and a downstream sequence of budC ORF. This fragment was introduced into the SC17(0)/RSF-Red-TER strain by the λ-Red method described in (1-3), to obtain a strain SC17(0)ΔbudABC::Km/RSF-Red-TER as a Km-resistant strain. It was confirmed by PCR using primers of SEQ ID NOS: 15 and 16 that the Km resistance gene was inserted in the SC17(0)ΔbudABC::Km/RSF-Red-TER strain so as to delete the budABC region ORF. Genome extracted from the SC17(0)ΔbudABC::Km/RSF-Red-TER strain was introduced into Pantoea ananatis Glu-producing strain NA1 by the genomic transfer described in (1-4), to obtain a strain NA1ΔbudABC::Km as a Km-resistant strain. It was confirmed by PCR using primers of SEQ ID NOS: 15 and 16 that the Km resistance gene was inserted in the NA1ΔbudABC::Km strain so as to delete the budABC region ORF.

### (3) L-glutamic acid production by P. ananatis NA1ΔbudABC::Km strain

P. ananatis NA1 strain was cultured on LBGM9 agar medium containing 12.5 mg/L Tet (tetracycline). P. ananatis NA1ΔbudABC::Km strain was cultured on LBGM9 agar medium containing 12.5 mg/L Tet and 40 mg/L Km. Cells collected from one-quarter of a plate were inoculated into 5 mL of Glu fermentation medium containing 12.5 mg/L Tet and cultured at 34°C for 24 hours. The amounts of residual sugar (RS) and L-glutamic acid in the culture broth were measured with a bioanalyzer BF5 (Oji Instrument).

The results are shown in Table 5. L-glutamic acid production was improved by deletion of the budABC genes. Thus, it was revealed that production of L-amino acids such as L-glutamic acid is improved by reducing the activity of BudA protein, BudB protein, and/or BudC protein.

**Table 5**

| Strain | NO. | RS (g/L) | OD620nm | Avg. | SD | Glu (g/L) | Avg. | SD | Yield (%) | Avg. | SD |
|---|---|---|---|---|---|---|---|---|---|---|---|
| P. ananatis NA1 | 1 | 0 | 18.5 | 18.1 | 0.38 | 10.5 | 10.3 | 0.19 | 35.0 | 34.3 | 0.64 |
| | 2 | 0 | 18.2 | | | 10.04 | | | 33.5 | | |
| | 3 | 0 | 17.6 | | | 10.36 | | | 34.5 | | |
| P. ananatis NA1△budABC::Km | 1 | 0 | 14.1 | 13.2 | 1.76 | 11.24 | 10.6 | 0.43 | 37.5 | 35.5 | 1.43 |
| | 2 | 0 | 14.7 | | | 10.42 | | | 34.7 | | |
| | 3 | 0 | 10.7 | | | 10.26 | | | 34.2 | | |
| Blank | | 30 | | | | 0.08 | | | | | |

### (4) Construction of P. ananatis NA1ΔGlcNDH::Km strain

PCR was carried out using genome of Pantoea ananatis SC17(0)hisD::(attL_{λ}-Km^{R}-attR_{λ}) (Joanna I Katashkina et al., Use of the lambda Red-recombineering method for genetic engineering of Pantoea ananatis, BMC Mol Biol, doi: 10.1186/1471-2199-10-34.) as a template in combination with primers of SEQ ID NOS: 17 and 18, to obtain an attL-Km-attR fragment for deletion of GlcNDH genes (PAJ_3461, PAJ 3462, and PAJ_3463), which fragment had been added with an upstream sequence of PAJ_3461 ORF and a downstream sequence of PAJ_3463 ORF. This fragment was introduced into the SC17(0)/RSF-Red-TER strain by the λ-Red method described in (1-3), to obtain a strain SC17(0)ΔGlcNDH::Km/RSF-Red-TER as a Km-resistant strain. It was confirmed by PCR using primers of SEQ ID NOS: 19 and 20 that the Km resistance gene was inserted in the SC17(0)ΔGlcNDH::Km/RSF-Red-TER strain so as to delete the GlcNDH region ORF. Genome extracted from the SC17(0)ΔGlcNDH::Km/RSF-Red-TER strain was introduced into Pantoea ananatis Glu-producing strain NA1 by the genomic transfer described in (1-4), to obtain a strain NA1ΔGlcNDH::Km as a Km-resistant strain. It was confirmed by PCR using primers of SEQ ID NOS: 19 and 20 that the Km resistance gene was inserted in the NA1ΔGlcNDH::Km strain so as to delete the GlcNDH region ORF.

### (5) L-glutamic acid production by P. ananatis NA1ΔGlcNDH::Km strain

P. ananatis NA1 strain was cultured on LBGM9 agar medium containing 12.5 mg/L Tet (tetracycline). P. ananatis NA1ΔGlcNDH::Km strain was cultured on LBGM9 agar medium containing 12.5 mg/L Tet and 40 mg/L Km. Cells collected from one-quarter of a plate were inoculated into 5 mL of Glu fermentation medium containing 12.5 mg/L Tet and cultured at 34°C for 24 hours. The amounts of residual sugar (RS) and L-glutamic acid in the culture broth were measured with a bioanalyzer BF5 (Oji Instrument).

The results are shown in Table 6. L-glutamic acid production was improved by deletion of the GlcNDH genes (PAJ_3461, PAJ_3462, and PAJ_3463). Thus, it was revealed that production of L-amino acids such as L-glutamic acid is improved by reducing the activity of PAJ_3461 protein, PAJ_3462 protein, and/or PAJ_3463 protein.

**Table 6**

| Strain | NO. | RS (g/L) | OD620nm | Avg. | SD | Glu (g/L) | Avg. | SD | Yield (%) | Avg. | SD |
|---|---|---|---|---|---|---|---|---|---|---|---|
| P. ananatis NA1 | 1 | 0 | 17.9 | 18.1 | 0.16 | 9.06 | 8.9 | 0.23 | 30.2 | 29.6 | 0.76 |
| | 2 | 0 | 18.3 | | | 9.02 | | | 30.1 | | |
| | 3 | 0 | 18.1 | | | 8.56 | | | 28.5 | | |
| P. ananatis NA1△GlcNDH::Km | 1 | 0 | 13.8 | 14.9 | 1.12 | 10.28 | 9.9 | 0.39 | 34.3 | 32.9 | 1.31 |
| | 2 | 0 | 14.4 | | | 10 | | | 33.3 | | |
| | 3 | 0 | 16.5 | | | 9.34 | | | 31.1 | | |
| Blank | | 30 | | | | 0.18 | | | | | |

### (6) Construction of P. ananatis NA1ΔGlcNDH strain

To remove the antibiotic resistance marker gene introduced into the chromosome, pMW-intxis-sacB(Spc) (WO2015/005405A1), a helper plasmid for expression of an int-xis gene of λ phage, was introduced into the gene-deletion strain obtained in (4) by electroporation method, and colonies were formed at 30°C using LB medium containing 100 mg/L Spc. Then, single colonies were isolated at 34°C using LB medium without Spc but containing 10% Sucrose, M9 minimal salts, and 1 mM IPTG, and a strain from which pMW-intxis-sacB(Spc) and the antibiotic resistance marker gene introduced into chromosome had been removed was obtained on the basis of sensitivity to antibiotics (Spc and antibiotic corresponding to the antibiotic resistance marker gene introduced into chromosome).

### (7) Construction of P. ananatis NA1ΔGlcNDHΔbudABC::Km strain

Genome extracted from the SC17(0)ΔbudABC::Km/RSF-Red-TER strain constructed in (2) was introduced into P. ananatis NA1ΔGlcNDH strain by the genomic transfer described in (1-4), to obtain a strain NA1ΔGlcNDHΔbudABC::Km as a Km-resistant strain. It was confirmed by PCR using primers of SEQ ID NOS: 15 and 16 that the Km resistance gene was inserted in the NA1ΔGlcNDHΔbudABC::Km strain so as to delete the budABC region ORF.

### (8) L-glutamic acid production by P. ananatis NA1ΔGlcNDHΔbudABC::Km strain

P. ananatis NA1 strain was cultured on LBGM9 agar medium containing 12.5 mg/L Tet (tetracycline). P. ananatis strains NA1ΔGlcNDH::Km, NA1ΔbudABC::Km, and NA1ΔGlcNDHΔbudABC::Km were each cultured on LBGM9 agar medium containing 12.5 mg/L Tet and 40 mg/L Km. Cells collected from one-quarter of a plate were inoculated into 5 mL of Glu fermentation medium containing 12.5 mg/L Tet and cultured at 34°C for 24 hours. The amounts of residual sugar (RS) and L-glutamic acid in the culture broth were measured with a bioanalyzer BF5 (Oji Instrument).

The results are shown in Table 7. L-glutamic acid production per cell was improved by deletion of the GlcNDH genes (PAJ_3461, PAJ 3462, and PAJ_3463) and deletion of the budABC genes. In addition, L-glutamic acid production per cell was further improved by a combination of deletion of the GlcNDH genes and deletion of the budABC genes as compared to when only the GlcNDH genes were deleted and when only the budABC genes were deleted. Thus, it was revealed that production of L-amino acids such as L-glutamic acid per cell is improved by combining a reduction in the activity of PAJ_3461 protein, PAJ_3462 protein, and/or PAJ_3463 protein with a reduction in the activity of BudA protein, BudB protein, and/or BudC protein.

**Table 7**

| Strain | NO. | RS (g/L) | OD620nm | Avg. | SD | Glu (g/L) | Avg. | SD | Yield (%) | Avg. | SD | Glu /OD |
|---|---|---|---|---|---|---|---|---|---|---|---|---|
| P. ananatis NA1 | 1 | 0 | 15.4 | 16.3 | 0.89 | 8.52 | 8.4 | 0.07 | 27.2 | 26.9 | 0.21 | 0.52 |
| | 2 | 0 | 16.0 | | | 8.42 | | | 26.9 | | | |
| | 3 | 0 | 17.5 | | | 8.36 | | | 26.7 | | | |
| P. ananatis NA1△GlcNDH::Km | 1 | 0 | 15.5 | 15.3 | 0.29 | 8.86 | 8.8 | 0.22 | 28.3 | 28.2 | 0.71 | 0.58 |
| | 2 | 0 | 14.8 | | | 9.1 | | | 29.1 | | | |
| | 3 | 0 | 15.5 | | | 8.56 | | | 27.3 | | | |
| P. ananatis NA1△budABC::Km | 1 | 0 | 13.4 | 13.4 | 0.05 | 9.32 | 9.4 | 0.03 | 29.8 | 29.9 | 0.11 | 0.70 |
| | 2 | 0 | 13.3 | | | 9.4 | | | 30.0 | | | |
| | 3 | 0 | 13.3 | | | 9.34 | | | 29.8 | | | |
| P. ananatis NA1△GlcNDH △budABC::Km | 1 | 0 | 13.1 | 12.5 | 0.95 | 9.66 | 9.5 | 0.15 | 30.9 | 30.5 | 0.47 | 0.77 |
| | 2 | 0 | 13.1 | | | 9.64 | | | 30.8 | | | |
| | 3 | 0 | 11.1 | | | 9.34 | | | 29.8 | | | |
| Blank | | 31.3 | | | | 0.18 | | | | | | |

### <Explanation of Sequence Listing>

### SEQ ID NOS:

1: Nucleotide sequence of budA gene of P. ananatis AJ13355
2: Amino acid sequence of BudA protein of P. ananatis AJ13355
3: Nucleotide sequence of budB gene of P. ananatis AJ13355
4: Amino acid sequence of BudB protein of P. ananatis AJ13355
5: Nucleotide sequence of budC gene of P. ananatis AJ13355
6: Amino acid sequence of BudC protein of P. ananatis AJ13355
7: Nucleotide sequence of PAJ_3461 gene of P. ananatis AJ13355
8: Amino acid sequence of PAJ_3461 protein of P. ananatis AJ13355
9: Nucleotide sequence of PAJ_3462 gene of P. ananatis AJ13355
10: Amino acid sequence of PAJ_3462 protein of P. ananatis AJ13355
11: Nucleotide sequence of PAJ_3463 gene of P. ananatis AJ13355
12: Amino acid sequence of PAJ_3463 protein of P. ananatis AJ13355
13-20: Primers

## Claims

1. A method for producing an L-amino acid, the method comprising:
culturing a bacterium belonging to the family Enterobacteriaceae and having an L-amino acid-producing ability in a culture medium to accumulate the L-amino acid in the culture medium and/or cells of the bacterium; and
collecting the L-amino acid from the culture medium and/or the cells,
wherein the L-amino acid is an L-amino acid of glutamate family, and
wherein the bacterium has one or more modifications selected from the modifications (A) to (F) shown below:
(A) modification of reducing the activity of a BudA protein;
(B) modification of reducing the activity of a BudB protein;
(C) modification of reducing the activity of a BudC protein;
(D) modification of reducing the activity of a PAJ_3461 protein;
(E) modification of reducing the activity of a PAJ_3462 protein; and
(F) modification of reducing the activity of a PAJ_3463 protein.

2. The method according to claim 1, wherein the bacterium at least has one or more modifications selected from the modifications (A) to (C).

3. The method according to claim 1 or 2, wherein the bacterium at least has the modifications (A) to (C).

4. The method according to any one of claims 1 to 3, wherein the bacterium at least has one or more modifications selected from the modifications (D) to (F).

5. The method according to any one of claims 1 to 4, wherein the bacterium at least has the modifications (D) to (F).

6. The method according to any one of claims 1 to 5, wherein the bacterium has the modifications (A) to (F).

7. The method according to any one of claims 1 to 6,
Wherein the activity of the BudA protein is reduced by reducing the expression of a budA gene and/or disrupting the budA gene;
Wherein the activity of the BudB protein is reduced by reducing the expression of a budB gene and/or disrupting the budB gene;
Wherein the activity of the BudC protein is reduced by reducing the expression of a budC gene and/or disrupting the budC gene;
Wherein the activity of the PAJ_3461 protein is reduced by reducing the expression of a PAJ_3461 gene and/or disrupting the PAJ_3461 gene;
Wherein the activity of the PAJ_3462 protein is reduced by reducing the expression of a PAJ_3462 gene and/or disrupting the PAJ_3462 gene; and/or
Wherein the activity of the PAJ_3463 protein is reduced by reducing the expression of a PAJ_3463 gene and/or disrupting the PAJ_3463 gene.

8. The method according to claim 7,
wherein the expression of the budA gene is reduced by modifying an expression control sequence of the budA gene;
wherein the expression of the budB gene is reduced by modifying an expression control sequence of the budB gene;
wherein the expression of the budC gene is reduced by modifying an expression control sequence of the budC gene;
wherein the expression of the PAJ_3461 gene is reduced by modifying an expression control sequence of the PAJ_3461 gene;
wherein the expression of the PAJ_3462 gene is reduced by modifying an expression control sequence of the PAJ_3462 gene; and/or
wherein the expression of the PAJ_3463 gene is reduced by modifying an expression control sequence of the PAJ_3463 gene.

9. The method according to any one of claims 1 to 8,
Wherein the activity of the BudA protein is reduced by deletion of the budA gene;
Wherein the activity of the BudB protein is reduced by deletion of the budB gene;
Wherein the activity of the BudC protein is reduced by deletion of the budC gene;
Wherein the activity of the PAJ_3461 protein is reduced by deletion of the PAJ_3461 gene;
Wherein the activity of the PAJ_3462 protein is reduced by deletion of the PAJ_3462 gene; and/or
Wherein the activity of the PAJ_3463 protein is reduced by deletion of the PAJ_3463 gene.

10. The method according to any one of claims 1 to 9,
wherein the BudA protein is the protein (1a), (1b), or (1c) shown below:
(1a) a protein comprising the amino acid sequence of SEQ ID NO: 2;
(1b) a protein comprising the amino acid sequence of SEQ ID NO: 2, but which includes substitution, deletion, insertion, and/or addition of 1 to 10 amino acid residues, and having acetolactate decarboxylase activity;
(1c) a protein comprising an amino acid sequence showing an identity of 90% or higher to the amino acid sequence of SEQ ID NO: 2, and having acetolactate decarboxylase activity;
wherein the BudB protein is the protein (2a), (2b), or (2c) shown below:
(2a) a protein comprising the amino acid sequence of SEQ ID NO: 4;
(2b) a protein comprising the amino acid sequence of SEQ ID NO: 4, but which includes substitution, deletion, insertion, and/or addition of 1 to 10 amino acid residues, and having acetolactate synthase activity;
(2c) a protein comprising an amino acid sequence showing an identity of 90% or higher to the amino acid sequence of SEQ ID NO: 4, and having acetolactate synthase activity;
wherein the BudC protein is the protein (3a), (3b), or (3c) shown below:
(3a) a protein comprising the amino acid sequence of SEQ ID NO: 6;
(3b) a protein comprising the amino acid sequence of SEQ ID NO: 6, but which includes substitution, deletion, insertion, and/or addition of 1 to 10 amino acid residues, and having (S,S)-butanediol dehydrogenase activity, meso-2,3-butandiol dehydrogenase activity, and/or diacetyl reductase activity;
(3c) a protein comprising an amino acid sequence showing an identity of 90% or higher to the amino acid sequence of SEQ ID NO: 6, and having (S,S)-butanediol dehydrogenase activity, meso-2,3-butandiol dehydrogenase activity, and/or diacetyl reductase activity;
wherein the PAJ_3461 protein is the protein (4a), (4b), or (4c) shown below:
(4a) a protein comprising the amino acid sequence of SEQ ID NO: 8;
(4b) a protein comprising the amino acid sequence of SEQ ID NO: 8, but which includes substitution, deletion, insertion, and/or addition of 1 to 10 amino acid residues, and having gluconate 2-dehydrogenase activity;
(4c) a protein comprising an amino acid sequence showing an identity of 90% or higher to the amino acid sequence of SEQ ID NO: 8, and having gluconate 2-dehydrogenase activity;
wherein the PAJ_3462 protein is the protein (5a), (5b), or (5c) shown below:
(5a) a protein comprising the amino acid sequence of SEQ ID NO: 10;
(5b) a protein comprising the amino acid sequence of SEQ ID NO: 10, but which includes substitution, deletion, insertion, and/or addition of 1 to 10 amino acid residues, and having gluconate 2-dehydrogenase activity;
(5c) a protein comprising an amino acid sequence showing an identity of 90% or higher to the amino acid sequence of SEQ ID NO: 10, and having gluconate 2-dehydrogenase activity; and/or
wherein the PAJ_3463 protein is the protein (6a), (6b), or (6c) shown below:
(6a) a protein comprising the amino acid sequence of SEQ ID NO: 12;
(6b) a protein comprising the amino acid sequence of SEQ ID NO: 12, but which includes substitution, deletion, insertion, and/or addition of 1 to 10 amino acid residues, and having gluconate 2-dehydrogenase activity;
(6c) a protein comprising an amino acid sequence showing an identity of 90% or higher to the amino acid sequence of SEQ ID NO: 12, and having gluconate 2-dehydrogenase activity.

11. The method according to any one of claims 1 to 10, wherein the bacterium is a Pantoea bacterium or an Escherichia bacterium.

12. The method according to any one of claims 1 to 11, wherein the bacterium is Pantoea ananatis or Escherichia coli.

13. The method according to any one of claims 1 to 12, wherein the L-amino acid of glutamate family is one or more L-amino acids selected from the group consisting of L-glutamic acid, L-glutamine, L-proline, L-arginine, L-citrulline, and L-ornithine.

14. The method according to any one of claims 1 to 13, wherein the L-amino acid of glutamate family is L-glutamic acid.

15. The method according to claim 13 or 14, wherein the L-glutamic acid is ammonium L-glutamate or sodium L-glutamate.
